# EUROPEAN PATENT APPLICATION

(11) **EP 4 032 466 A1**
(43) Date of publication of application: **27.07.2022**
(21) Application number: 21218390.9
(22) Date of filing: 30.12.2021
(51) Int. Cl.: A61B 5/00, A61B 5/01, A61F 7/00

(54) **THERMAL IMAGING DETECTION**

(30) Priority: 05.01.2021 US 202117142023
(71) Applicant: Quest Medical, Inc., Allen, TX 75002 (US)
(72) Inventor: ALBERTSEN, Jeffrey, Allen, TX 75002 (US); NISHTALA, Vasu, Allen, TX 75002 (US); MART, Robert, Allen, TX 75002 (US); PARNIS, Steven, Allen, TX 75002 (US)
(74) Representative: Barker Brettell LLP

(57) **Abstract**

The present disclosure is related to an apparatus, system, and method of thermal detection during surgical or medical procedures. These procedures can include open-heart surgery with a pump (114; 214; 314; 414; 514; 614) connected to a thermal detection unit (102; 202; 302; 402; 502; 602) and a computing device (106; 206; 306; 406; 506; 606) for sending and receiving signals to control the pump(114; 214; 314; 414; 514; 614). Detecting thermal changes with a thermal detection unit (102; 202; 302; 402; 502; 602) in an organ or tissue (320) and controlling a pump (114; 214; 314; 414; 514; 614) based on the analysis of the image generated with a thermal imager unit (104; 204; 304; 404; 504; 604) from the detected thermal changes. The thermal detection system (100; 200; 300; 400; 500; 600) has a detection (102; 202; 302; 402; 502; 602) and imaging unit (104; 204; 304; 404; 504; 604), coupled to a computing device (106; 206; 306; 406; 506; 606) for processing and analysis and utilized as a part of a control system for a perfusion pump (114; 214; 314; 414; 514; 614).

## Description

### BACKGROUND

### Cross-Reference to Related Applications

This Application claim priority benefit of U.S. Application No. 17/142,023 filed January 5, 2021, the disclosure of which is hereby incorporated by reference.

### Technical Field

The present disclosure relates to thermal detection. More particularly, and not by way of limitation, the present disclosure is directed to an apparatus, system, and method for thermal detection during medical operations.

### Description of Related Art

In the medical field, temperature measurements are some of the most critical and most often utilized measurements. From fevers, hypothermia, or during surgical operations, the temperature of specific body parts, organs, or tissue can be critical. However, measurement is difficult during operations or surgical procedures. In particular, during open-heart procedures, there is a need to monitor the perfusion of a cardioplegia drug that slows or temporarily stops the heart. If there are any portions of the heart that do not receive the proper dosing of the cardioplegia drug, there is a risk of damage or injury during the surgery.

Thermal imaging has been utilized for many industrial/commercial applications such as leak diagnostics, heat exchangers, HVAC, firefighting, insulation, or security. However, in the medical field, the uses have been limited. A growing list of medical applications includes identifying areas of inflammation, detection of tumor margins, and in peripheral blood flow, cardiovascular disease, and breast cancer diagnostics. These medical applications can include unique needs as to the particular space and imaging requirements. It would be advantageous to have an apparatus, system, or method for thermal detection that overcomes the disadvantages of the prior art.

### BRIEF SUMMARY

The present disclosure is related to thermal detection during surgical or medical procedures. These procedures can include open-heart surgery with a pump connected to a thermal detection unit and a computing device to send and receive signals to control the pump. In yet another embodiment, a method of thermal detection includes detecting thermal changes in an organ or tissue and controlling a pump based on the analysis of the image generated from the detected thermal changes. Another embodiment can be a thermal detection system having a detection and imaging unit, coupled to a computing device for processing and analysis, and utilized as a part of a control system for a perfusion pump.

### BRIEF DESCRIPTION OF THE DRAWINGS

The novel features believed characteristic of the disclosure are set forth in the appended claims. The disclosure itself, however, as well as a preferred mode of use, further objectives, and advantages thereof, will be best understood by reference to the following detailed description of illustrative embodiments when read in conjunction with the accompanying drawings, wherein:
**FIG. 1** is a block diagram view of a thermal imaging detection system;
**FIG. 2A** is a block diagram illustration of a thermal imaging detection system with an integrated light unit;
**FIG. 2B** is a side view illustration of a medical facility with a thermal imaging detection system with an integrated light unit;
**FIG. 2C** is an illustration of a light unit with an incorporated detection unit;
**FIG. 3** is a block diagram illustration of a thermal imaging detection system with an integrated feedback control loop;
**FIG. 4** is an illustration of data capture from a thermal imaging detection system;
**FIG. 5** is an illustration of data capture from a thermal imaging detection system with an integrated feedback loop;
**FIG. 6** is an illustration of data capture from a thermal imaging detection system with an integrated feedback loop after the execution of a feedback operation;
**FIG. 7** is an illustration of an operational flow for thermal imaging detection;
**FIG. 8** is an illustration of an operational flow for thermal imaging detection from a surgical or medical light; and
**FIG. 9** is an illustration of an operational flow for thermal imaging detection with a feedback loop.

### DETAILED DESCRIPTION

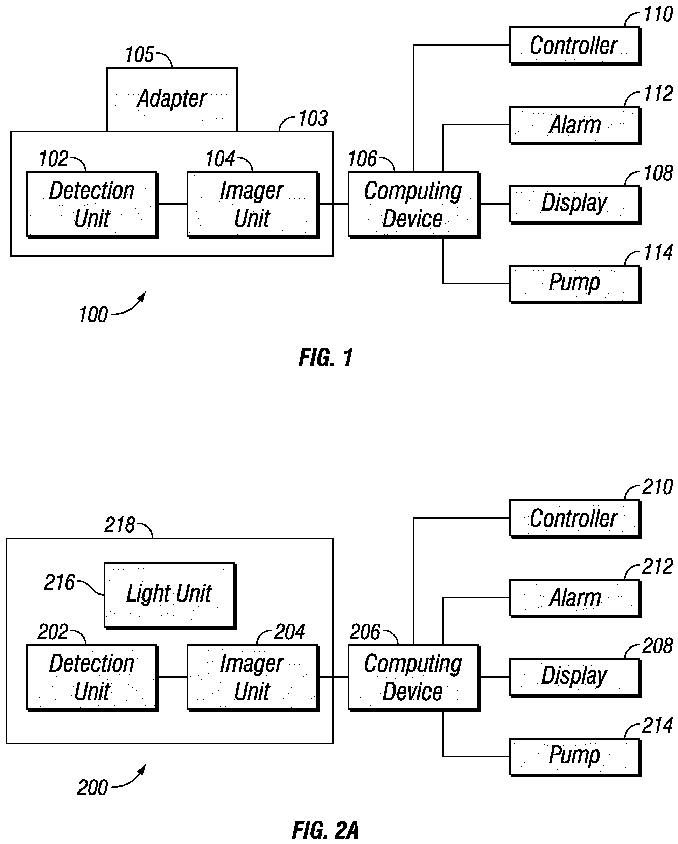

Embodiments of the disclosure will now be described. FIG. 1 is a block diagram view of a thermal imaging detection system 100. The thermal imaging detection system 100 can be configured to provide thermal imaging for a doctor's viewing during a procedure or related medical operation. The thermal imaging detection system can be hand-held, attached to other available equipment, or attached to a dedicated pole. In at least one example, the thermal imaging detection system 100 may be utilized within a medical or surgical light to provide overhead thermal imaging during a procedure or a related medical operation. Doctors and medical personnel often provide fluids to patients to increase or decrease temperatures for specific operations and portions of organs or tissue. For example, during procedures or related medical operations related to the heart, fluids are perfused into the heart muscle that can slow or speed up the beating of the heart muscle, and the perfusion can be monitored by the changes in a thermal gradient of the tissue. As perfusion occurs, the temperature gradient or change in the tissue can be detected and utilized to determine if proper perfusion of medications or other fluids is occurring properly. If the perfusion is not occurring properly, an alarm or other indication may be provided to individuals near the procedure or related medical operation in a manner that causes individuals to monitor the patient with greater detail.

The thermal imaging detection system 100 may utilize a detection unit 102 for capturing data regarding thermal changes to organs or tissues affected by temperature changes or gradient changes during a procedure or related medical operation. The detection unit 102 may allow for the detection of temperature changes or gradients as small as 0.001 degrees Fahrenheit, or its equivalent temperature in Celsius or Kelvin. In at least one example, detection can occur in a range from negative thirty (-30) degrees Fahrenheit, to one hundred twenty-five (125) degrees Fahrenheit, or equivalent temperatures in Celsius (-35 to 50 °C) or Kelvin. In some examples, the temperature range could extend from negative fifty (-50) degrees Fahrenheit, to one-hundred fifty (150) degrees Fahrenheit, or equivalent temperatures in Celsius or Kelvin. The data or data sets provided from the detection unit 102 may include coordinates, temperature readings, time stamps, temperature changes over time, discrete temperature, other additional data or information, comparisons, or combinations thereof. In at least one example, multiple detection unit(s) 102 may be coupled together or multiplexed for the transmission of data or data sets to allow for multi-dimensional imaging to occur. For example, at least four detection unit(s) 102 could be coupled or fastened to four separate points that are distal from one another along a surgical or medical light utilized in procedures or other related medical operations. Other housings may also be utilized for the detection unit(s) 102 and/or imager unit(s) 104. These other housings may be an enclosure fixed to a specific location of a medical or surgical suite or other medical facility, allowing doctors or other medical personnel to view organs and tissues at more than one level to determine if perfusion or other monitored conditions or operations are occurring correctly or if changes to the procedure, operation, medicines, or other activities need to occur. In some examples, the data or data set generated by the detection unit(s) 102 may be stored in memory or storage as part of the detection unit(s) 102 or transmitted in real- or near real-time. The memory or storage may be part of a system on a chip (SOC), permeant units directly coupled to the detection unit(s) 102, or removable units that are received by the detection unit(s) 102.

The detection unit 102 and/or imager unit 104 may be housed within a handheld unit 103. The handheld unit 103 can allow medical professionals to monitor specific tissue, organs, or locations for temperature changes. In at least one example, the handheld unit 103 may also be utilized to internal temperature measurements of a patient or used in other detection or imager units. The handheld unit 103, in at least one embodiment, can have an adaptor 105 for coupling and/or storing the handheld unit 103 to a specific location. The adaptor 105 can allow for the coupling of the handheld unit 103 to devices, equipment, or locations in a medical center, surgical suite, operating room, and/or hospital. For example, the handheld unit 103 may be mounted through the adaptor 105 to a pole configured for attachment to a hospital bed or surgical gurney. Other examples may include a specialized pole capable of being attached to a surgical light and being flexible for positioning over a patient.

The data or data sets recorded or transmitted by the detection unit(s) 102 can be provided to an imager unit 104. In at least one example, the imager unit 104 may be incorporated as part of the detection unit(s) 102 or a computing device 106. In at least one embodiment, the detection unit 102 may be called a thermal detector, and the imager unit 104 may be called a thermal imager. The imager unit 104 can take the data or data sets provided by the detection unit(s) 102 for conversion into viewable images, video, or other displayable formats. In at least one embodiment, the imager unit 104 can generate two- or three-dimensional images or video from the data or data sets. These images or video may be displayed through a computing device 106, or through other devices such as, but not limited to, televisions, displays, mobile devices, tablets, phones, Personal Digital Assistants (PDAs), digital watches, smart-watches, or other devices capable of allowing viewing of information or data provided to it. In at least one example, the imager unit 104 may also overlay additional data or information, such as, but not limited to, zones, gridlines, highlights, color maps, legends, time or date information, patient information, hospital information, doctor or medical staff information, and/or other valuable information that may be received or processed by the imager unit 104. In other examples, the additional data or information may be provided by a computing device 106, a database connected to the computing device through a computer network, and/or other devices coupled to a computer network capable of transmitting data or information to the computing device 106 or the imager unit 104.

The computing device 106 can be utilized to process, analyze, network, and/or communicate information or data from the various device connected, coupled, or otherwise interfaced with the computing device 106. In at least one embodiment, the computing device 106 is connected to the imager unit 104, a display 108, and a user interface 110. The computing device 106 can allow for the processing of images or video from the imager unit(s) 104. For example, the imager unit 104 provides a thermal gradient image to the computing device 106 for processing and analysis. The computing device 106 creates a set of sections or zones to be reviewed for specific temperature changes or gradients. If a section or zone is outside of the specific range of temperature changes or gradients, then an alert can be generated. In some examples, the temperature changes or gradients may need to be outside a specified range of temperature changes or gradients, and an alert can be generated if the temperature change or gradient is within the specified range. The computing device 106 may also allow for the transmission of data, information, analysis, or data sets over a computer network to various databases, electronic devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation. Data or data sets from the detection unit 102 may be compared to the temperature readings or data from the scan prior to the procedure or medical operation. Alerts may include sounds, notifications, emails, visual indicators, or other manner of capturing the attention of a medical professional. For example, a controller, computing device, or user interface 110 may allow a medical professionals to input or provide preferences and/or settings for the analysis of the data, data sets, images, videos, or other information provided to the computing device 106. In some examples, a controller, computing device, or user interface 110 may allow for signals, alerts, or other information to be received and acted upon to trigger additional activities or operations. In this example, a controller 110 may receive an alert, which triggers the controller to activate additional medications or requests to a pharmacy for additional medications to be available. The controller or user interface 110 can be a contrast controller to increase or decrease contrast materials to a patient or through intermediate devices to the patient. A contrast controller may also interact with or through a computing device 106 to adjust the contrast of images, video, or data received from the detection unit 102 or imager unit 104. For example, the contrast controller may adjust the detected temperature range or the scale of the temperature ranges. Further to this example, the ranges may be adjusted from a linear scale to an exponential scale or other scales as desired by the user or medical professional.

The same alert received by the controller 110 may also be received by an alarm 112 or similar device that can attract the attention of a medical professional or other individual that should be alerted to a change in a detected condition. In some examples, the alert may be provided to a display 108 that can allow medical professionals or other users to review the data provided and act on the information accordingly. For example, a medical professional upon receiving the alert may want to have a computing device 106 or controller 110 modify the settings of a pump 114 that is actively providing the fluid being perfused within the organ or tissue.

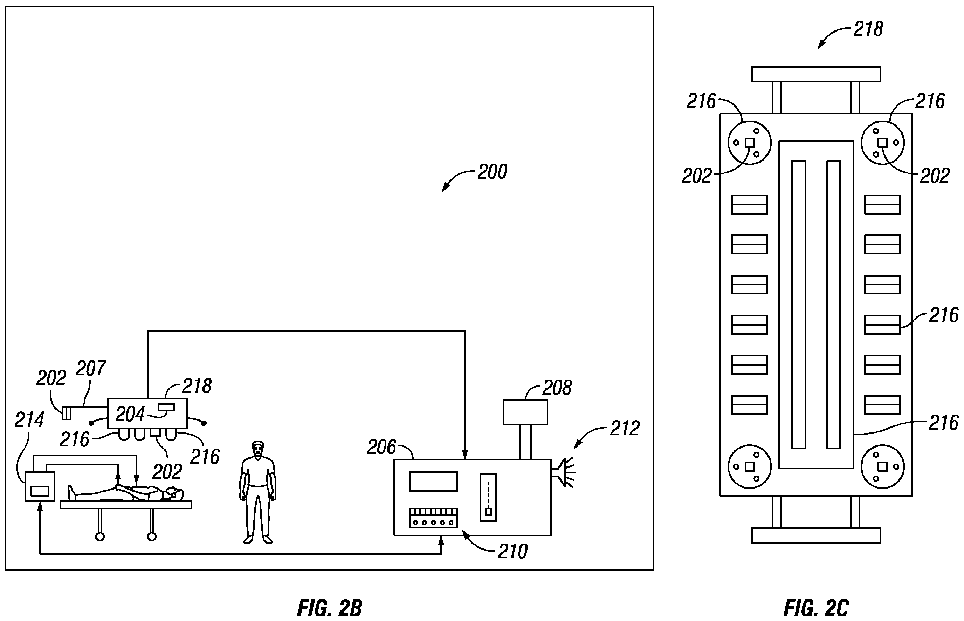

FIG. 2A is a block diagram illustration of a thermal imaging detection system 200 with integrated light unit 216. FIG. 2B is a side view illustration of a medical facility with a thermal imaging detection system 200 with integrated light unit 216. FIG. 2C is an illustration of a light unit 216 with incorporated detection unit 202. With reference to FIGs. 2A, 2B, and 2C, the thermal imaging detection system 200 can be configured to provide thermal imaging for viewing by a doctor during a procedure or related medical operation. In at least one embodiment, the thermal imaging detection system 200 is utilized within a medical or surgical light to provide overhead thermal imaging during a procedure or related medical operation. Doctors and medical personnel often provide fluids to patients to increase or decrease temperatures for specific operations and portions of organs or tissue. For example, during procedures or medical operations related to the heart, fluids are perfused into the heart muscle that can slow or speed up the beating of the heart muscle, and the perfusion can be monitored by the changes in a thermal gradient of the tissue. As perfusion occurs, the temperature gradient or change in the tissue can be detected and utilized to determine if perfusion of medications or other fluids is occurring properly. If the perfusion is not occurring properly, an alarm or other indication may be provided to individuals near the procedure or related medical operation in a manner that causes individuals to monitor the patient with greater detail.

The thermal imaging detection system 200 may utilize a detection unit 202 for capturing data regarding thermal changes to organs or tissues affected by temperature changes or gradient changes during a procedure or related medical operation. The detection unit 202 may allow for the detection of temperature changes or gradients as small as 0.001 degrees Fahrenheit, or its equivalent temperature in Celsius or Kelvin. In at least one example, detection can occur in a range from negative thirty (-30) degrees Fahrenheit, to one hundred twenty-five (125) degrees Fahrenheit, or equivalent temperatures in Celsius (-35 to 50 °C) or Kelvin. In some examples, the temperature range could extend from negative fifty (-50) degrees Fahrenheit, to one-hundred fifty (150) degrees Fahrenheit, or equivalent temperatures in Celsius or Kelvin. The data or data sets provided from the detection unit 202 may include coordinates, temperature readings, time stamps, temperature changes over time, discrete temperature, other additional data or information, comparisons, or combinations thereof. In at least one example, multiple detection unit(s) 202 may be coupled together or multiplexed for the transmission of data or data sets, to allow for multi-dimensional imaging to occur. For example, at least four detection unit(s) 202 could be coupled or fastened to four separate, distal points along a surgical or medical light 218 utilized in procedures or other related medical operations, allowing doctors or other medical personnel to view organs and tissues at more than one level to determine if perfusion or other monitored conditions or operations are occurring correctly or if changes to the procedure, operation, medicines, or other activities need to occur. In some examples, the data or data set generated by the detection unit(s) 202 may be stored in memory or storage as part of the detection unit(s) 202 or transmitted in real- or near real-time. The memory or storage may be part of a system on a chip (SOC), permeant units directly coupled to the detection unit(s) 202, or removable units that are received by the detection unit(s) 202.

The data or data sets recorded or transmitted by the detection unit(s) 202 can be provided to an imager unit 204. The imager unit 204 and the detection unit(s) 202 can be incorporated within a surgical or medical light 218, along with light units 216. In at least one embodiment, the detection unit 202 may be called a thermal detector, and the imager unit 204 may be called a thermal imager. In some examples, the light units 216 may have the detection unit(s) 202 integrated within them. For example, a light unit 216 may include three light-emitting diodes, bulbs, or other light sources with the detection unit 202 centrally arranged around them. The light unit 216 may have a specialized pole 207 that allows for the positioning of a detection unit 202, or an imager unit 204 at a position remote from the light unit 216. In at least one embodiment, multiple detection unit(s) 202 will be positioned in multiple locations within the surgical or medical light 218 to allow for multi-dimensional data or data sets, such as but not limited to, three-dimensional data or data sets to be generated. An imager unit 204 can be assigned or connected to individual detection unit(s) 202 or received a multiplexed or multiple streams of data from multiple detection units 202.

The imager unit 204 can take the data or data sets provided by the detection unit(s) 202 for conversion into viewable images, video, or other displayable formats. In at least one embodiment, the imager unit 204 can generate two- or three-dimensional images or video from the data or data sets. These images or video may be displayed through a computing device 206, or through other devices such as, but not limited to, televisions, displays, mobile devices, tablets, phones, Personal Digital Assistants (PDAs), digital watches, smart-watches, or other devices capable of allowing viewing of information or data provided to it. In at least one example, the imager unit 204 may also overlay additional data or information, such as, but not limited to, zones, gridlines, highlights, color maps, legends, time or date information, patient information, hospital information, doctor or medical staff information, and/or other valuable information that may be received or processed by the imager unit 204. In other examples, the additional data or information may be provided by a computing device 206, a database connected to the computing device through a computer network, and/or other devices coupled to a computer network capable of transmitting data or information to the computing device 206 or the imager unit 204.

The computing device 206 can be utilized to process, analyze, network, and/or communicate information or data from the various device connected, coupled, or other interfaced with the computing device 206. In at least one embodiment, the computing device 206 is connected to the imager unit 204, a display 208, and a user interface 210. The computing device 206 can allow for the processing of images or video from the imager unit(s) 204. For example, a thermal gradient image is provided from the imager unit 204 to the computing device 206 for processing and analysis. The computing device 206 creates a set of sections or zones to be reviewed for specific temperature changes or gradients. If a section or zone is outside of the specific range of temperature changes or gradients, then an alert can be generated. In some examples, the temperature changes or gradients may need to be outside a specified range of temperature changes or gradients, and an alert can be generated if the temperature change or gradient is within the specified range. The computing device 206 may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation. Data or data sets from the detection unit 102 may be compared to the temperature readings or data from the scan prior to the procedure or medical operation. Alerts may include sounds, notifications, emails, visual indicators, or other manner of capturing the attention of a medical professional. For example, a controller, computing device, or user interface 210 may allow for a medical professional to input or provide preferences and/or settings for the analysis of the data, data sets, images, videos, or other information provided to the computing device 206. In some examples, a controller, computing device, or user interface 210 may allow for signals, alerts, or other information to be received and acted upon to trigger additional activities or operations. In this example, a controller 210 may receive an alert, which triggers the controller to activate additional medications or requests to a pharmacy for additional medications to be available.

The same alert received by the controller 210 may also be received by an alarm 212 or similar device that can attract the attention of a medical professional or other individual that should be alerted to a change in a detected condition. In some examples, the alert may be provided to a display 208 that can allow medical professionals or other users to review the data provided and act on the information accordingly. For example, a medical professional, upon receiving the alert, may want to have a computing device 206 or controller 210 modify the settings of a pump 214 that is actively providing the fluid being perfused within the organ or tissue.

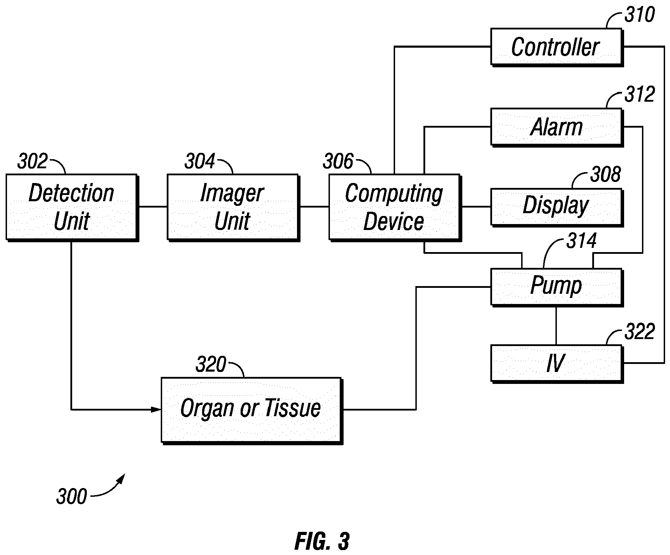

FIG. 3 is a block diagram illustration of a thermal imaging detection system 300 with an integrated feedback control loop. The thermal imaging detection system 300 can be configured to provide thermal imaging for a doctor's viewing during a procedure or related medical operation. In some examples, the thermal imaging detection system 300 can provide a feedback loop that allows for the control or recommended control of a pump 314 for delivering IV, medicines, or other fluids to a patient or specific organs or tissue 320.

Doctors and medical personnel often provide fluids to patients to increase or decrease temperatures for specific operations and portions of organs or tissue. For example, during procedures or related medical operations related to the heart, fluids are perfused into the heart muscle that can slow or speed up the beating of the heart muscle, and the perfusion can be monitored by the changes in a thermal gradient of the tissue. A heart that slows down will have a temperature change or gradient difference from normal heart rhythms, in addition to the change in temperature or temperature gradient from the perfused fluids. As perfusion occurs, the temperature gradient or change in the tissue can be detected and utilized to determine if proper perfusion of medications or other fluids is occurring. If the perfusion is not occurring properly, an alarm or other indication may be provided to individuals near the procedure or related medical operation in a manner that causes individuals to monitor the patient with greater detail. In some examples, if perfusion is not occurring properly, a feedback loop of information or data can be analyzed to determine if the amount of fluid being perfused is proper or should be changed. While this can be done automatically, a doctor or other medical professional may trigger the change after reviewing a recommendation from the thermal imaging detection system 300.

The thermal imaging detection system 300 may utilize a detection unit 302 for capturing data regarding thermal changes to organs or tissues 320 affected by temperature changes or gradient changes during a procedure or related medical operation. The detection unit 302 may allow for the detection of temperature changes or gradients as small as 0.001 degrees Fahrenheit, or its equivalent temperature in Celsius or Kelvin. In at least one example, detection can occur in a range from negative thirty (-30) degrees Fahrenheit, to one hundred twenty-five (125) degrees Fahrenheit, or equivalent temperatures in Celsius (-35 to 50 °C) or Kelvin. In some examples, the temperature range could extend from negative fifty (-50) degrees Fahrenheit, to one-hundred fifty (150) degrees Fahrenheit, or equivalent temperatures in Celsius or Kelvin. The data or data sets provided from the detection unit 302 may include coordinates, temperature readings, time stamps, temperature changes over time, discrete temperature, other additional data or information, comparisons, or combinations thereof. In at least one example, multiple detection unit(s) 302 may be coupled together or multiplexed for the transmission of data or data sets to allow for multi-dimensional imaging to occur. For example, at least four detection unit(s) 302 could be coupled or fastened to four separate distal points along a surgical or medical light utilized in procedures or other related medical operations, allowing doctors or other medical personnel to view organs and tissues at more than one level to determine if perfusion or other monitored conditions or operations are occurring correctly or if changes to the procedure, operation, medicines, or other activities need to occur. In some examples, the data or data set generated by the detection unit(s) 302 may be stored in memory or storage as part of the detection unit(s) 302 or transmitted in real- or near real-time. The memory or storage may be part of a system on a chip (SOC), permeant units directly coupled to the detection unit(s) 302, or removable units that are received by the detection unit(s) 302. In some examples, the detection unit(s) 302 or imager unit 304 may gather information regarding the ambient temperature of a room or area where the thermal image detection system 300 is being utilized.

The data or data sets recorded or transmitted by the detection unit(s) 302 can be provided to an imager unit 304. In at least one example, the imager unit 304 may be incorporated as part of the detection unit(s) 302 or a computing device 306. In at least one embodiment, the detection unit 302 may be called a thermal detector, and the imager unit 304 may be called a thermal imager. The imager unit 304 can take the data or data sets provided by the detection unit(s) 302 for conversion into viewable images, video, or other displayable formats. In at least one embodiment, the imager unit 304 can generate two- or three-dimensional images or video from the data or data sets. These images or video may be displayed through a computing device 306, or through other devices such as, but not limited to, televisions, displays, mobile devices, tablets, phones, Personal Digital Assistants (PDAs), digital watches, smart-watches, or other devices capable of allowing viewing of information or data provided to it. In at least one example, the imager unit 304 may also overlay additional data or information, such as, but not limited to, zones, gridlines, highlights, color maps, legends, time or date information, patient information, hospital information, doctor or medical staff information, and/or other valuable information that may be received or processed by the imager unit 304. In other examples, the additional data or information may be provided by a computing device 306, a database connected to the computing device through a computer network, and/or other devices coupled to a computer network capable of transmitting data or information to the computing device 306 or the imager unit 304.

The computing device 306 can be utilized to process, analyze, network, and/or communicate information or data from the various device connected, coupled, or other interfaced with the computing device 306. In at least one embodiment, the computing device 306 is connected to the imager unit 304, a display 308, and a user interface 310. The computing device 306 can allow for the processing of images or video from the imager unit(s) 304. For example, a thermal gradient image is provided from the imager unit 304 to the computing device 306 for processing and analysis. The computing device 306 creates a set of sections or zones to be reviewed for specific temperature changes or gradients. If a section or zone is outside of the specific range of temperature changes or gradients, then an alert can be generated. In some examples, the temperature changes or gradients may need to be outside a specified range of temperature changes or gradients, and an alert can be generated if the temperature change or gradient is within the specified range. The computing device 306 may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation. Data or data sets from the detection unit 302 may be compared to the temperature readings or data from the scan prior to the procedure or medical operation. Alerts may include sounds, notifications, emails, visual indicators, or other manner of capturing the attention of a medical professional. For example, a controller, computing device, or user interface 310 may allow for a medical professional to input or provide preferences and/or settings for the analysis of the data, data sets, images, videos, or other information provided to the computing device 306. The controller or user interface 310 may be utilized to control or receive signals from a fluid dispensing system 322. The fluid dispensing system 322 can be an IV or fluid control system that can utilize needleless connectors, manifolds, extension, or administration sets having check valves, roller clamps, filters, ball shutoffs, drop or drip control, or other administration tools. In some examples, a controller, computing device, or user interface 310 may allow for signals, alerts, or other information to be received and acted upon to trigger additional activities or operations. In this example, a controller 310 may receive an alert, which triggers the controller to activate additional medications or requests to a pharmacy for additional medications to be available. In yet another example, the fluid dispensing system 322 may provide additional fluids or combinations of fluids to the pump 314.

The same alert that is received by the controller 310 may also be received by an alarm 312 or similar device that can attract the attention of a medical professional or other individual that should be alerted to a change in a detected condition. In some examples, the alert may be provided to a display 308 that can allow medical professionals or other users to review the data provided and act on the information accordingly. For example, a medical professional, upon receiving the alert, may want to have a computing device 306 or controller 310 modify the settings of a pump 314 that is actively providing the fluid being perfused within the organ or tissue. One example of a pump 314 can be a Quest Medical^{®} MPS3 Myocardial Protection System or similar systems, that can include monitoring of the temperature of the fluid being provided to the patient, multiple pumping units within the pump 314 to allow for fluid and blood to be pumped through the pump 314 in at least one example. In some examples, the pump 314 may also include information regarding the cardiac condition of a patient, such as a cardioplegia ratio, potassium, ECG, blood pressure, flow rates, and other information regarding the patient or the specific organs or tissues 320.

In at least one example, temperature gradients or changes are utilized to ascertain cardioplegia (CPG) distribution for effective cardioprotection or protection of the myocardium. The determination of the cardioplegia distribution allows for intra-operative decisions on cardioplegia delivery based on routes of delivery such as but not limited to antegrade, retro or simulgrade, flow and pressure rates or time of delivery to achieve optimal distribution to ensure optimal protection of the heart. This allows users or medical professionals to customize cardioplegia delivery for specific underlying diseases, conditions, or patient anatomies. Users or medical professionals can utilize the presentation of images, videos, data or information, and/or the processing or analysis to provide decision assistance for the various parameters, conditions, or rules to provide to a fluid dispensing system or pump through an open loop system. For example, a representation through a temperature or heat map can allow for surface or volume distribution of cardioplegia through a heart and allow a user or medical processional to make adjustments to the delivery, delivery method, and/or delivery positioning to provide optimal delivery. Alternatively, the fluid dispensing system or pump may be controlled or auto regulated in a closed loop system based on the various parameters, conditions, or rules. For example, the flow rate of a cardioplegia can be controlled or auto regulated in the closed loop system.

In at least one embodiment, the computing device 306 is connected with the controller or user interface 310, the alarm 312, and the pump 314. The alarm 312 may also be coupled to the pump 314 to allow it to trigger alarms, alerts, notifications, or otherwise attracting the attention of a medical professional or other user based on information or signals from the computing device 306 or the pump 314. The pump 314 can receive fluids from a fluid dispensing system 322 that can be perfused within a blood supply pumped through the pump 314 or directly to the tissue or organ 320. The pump 314 may adjust the pumping operations, perfusion, or other operations based on signals from the computing device 306 or other instructions received from a medical professional or other user.

In at least one example, the computing device 306 can be coupled directly or indirectly to the detection unit 302 and/or the image unit 304, and the computing device 306 provides the image, video, data or information to a display 308 connected directly to the computing device 306. In some examples, the detection unit 302 and/or the image unit 304 can be coupled to the computing device 306 that is coupled to a user interface 310, which can be, but is not limited to, an operating room or surgical suite monitor system. Yet, in other examples, the computing device 306 can be coupled directly or indirectly to the detection unit 302 and/or the image unit 304, and the computing device 306 provides the image, video, data or information to a display 308 connected through a network or wireless connection to the computing device 306. Similarly, the image, video, data or information provided from the computing device 306, via the detection unit 302 and/or the image unit 304 may be provided to the fluid dispensing system 322 and/or the pump 314 either of which may have a display or monitor to provide the visual representations.

The computing device 306, in at least one example, can be configured to record or log images, video, or representations of data from the detection unit 302 and/or imager unit 304. Similarly, the images, video, or representations of data from the detection unit 302, and/or imager unit 304 may be played back by the computing device 306 to other devices coupled to the computing devices 306. Data logging of images, video, data or information from the detection unit 302 and/or imager unit 304, may allow for data and/or information to be gathered separated from personal information of a patient for analysis and further improvement of processing, algorithms, and/or analysis of image, video, data or information. In at least one embodiment, a user or medical professional can select data points, images, or video to determine discrete, average, maximum, minimum, and/or range of temperature at discrete moments in time or over a specified timer period. The computing device 306 may also allow for alerts or notification to be generated when temperatures are over or under a discreate or average temperature, over or under a specified temperature for a period of time or exceeded or not exceeded a specified temperature at discrete times, or over a period of time. In at least one embodiment, the computing device 306 can be utilized for a control system that may comprise the pump 314, fluid dispensing system 322, alarm 312, and/or controller or user interface 310. The control system can allow for clinical intervention if a patient's condition does not comply with normal or specified ranges. The clinical intervention can be activated by a doctor or medical professional based on detected temperatures or temperature changes. The control system can provide a feedback loop of information to determine if changes made by the control system or by a user are having the desired effects or if a different intervention may be desired.

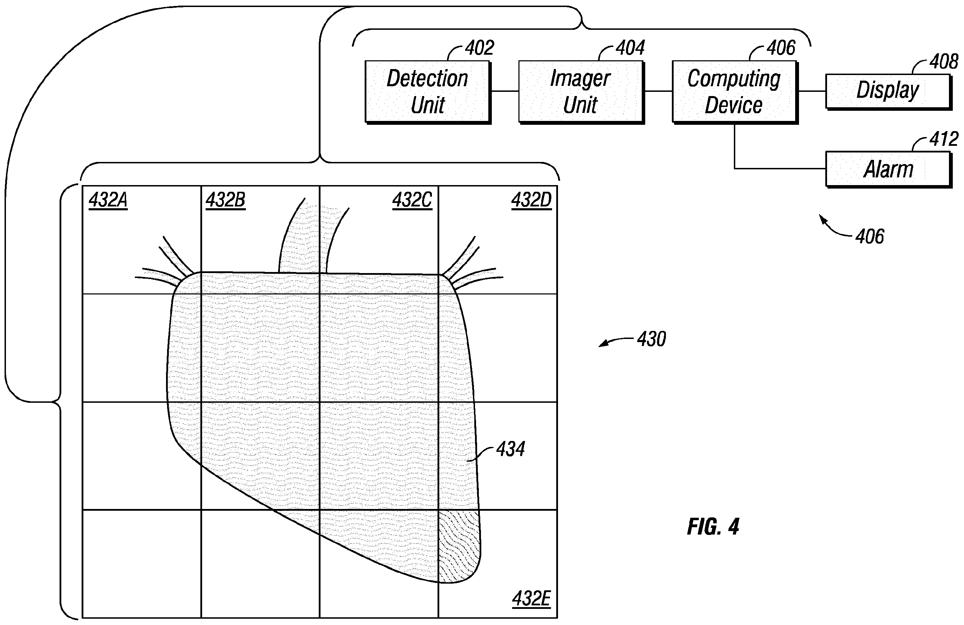

FIG. 4 is an illustration of data capture 430 from a thermal imaging detection system 400. The detection unit 402 of the thermal imaging detection system 400 can allow for a data capture 430 to occur. It should be noted, while data capture 430 is shown as an image, the data capture 430 may be processed by the imager unit 404 and/or the computing device 406 in order to show all of what is represented. In at least one embodiment, the detection unit 402 may be called a thermal detector, and the imager unit 404 may be called a thermal imager.

The thermal imaging detection system 400 may utilize a detection unit 402 for capturing data regarding thermal changes to organs or tissues affected by temperature changes or gradient changes during a procedure or related medical operation. The detection unit 402 may allow for the detection of temperature changes or gradients as small as 0.001 degrees Fahrenheit, or its equivalent temperature in Celsius or Kelvin. In at least one example, detection can occur in a range from negative thirty (-30) degrees Fahrenheit, to one hundred twenty-five (125) degrees Fahrenheit, or equivalent temperatures in Celsius (-35 to 50 °C) or Kelvin. In some examples, the temperature range could extend from negative fifty (-50) degrees Fahrenheit, to one-hundred fifty (150) degrees Fahrenheit, or equivalent temperatures in Celsius or Kelvin. The data or data sets provided from the detection unit 402 may include coordinates, temperature readings, time stamps, temperature changes over time, discrete temperature, other additional data or information, comparisons, or combinations thereof. In at least one example, multiple detection unit(s) 402 may be coupled together or multiplexed for the transmission of data or data sets, to allow for multi-dimensional imaging to occur. For example, at least four detection unit(s) 402 could be coupled or fastened to four separate points that are distal from one another along a surgical or medical light utilized in procedures or other related medical operations. Other housings may also be utilized for the detection unit(s) 402, and/or imager unit(s) 404. These other housings may be an enclosure fixed to a specific location of a medical or surgical suite or other medical facility. This can allow doctors or other medical personnel to view organs and tissues at more than one level to determine if perfusion or other monitored conditions or operations are occurring correctly or if changes to the procedure, operation, medicines, or other activities need to occur. In some examples, the data or data set generated by the detection unit(s) 402 may be stored in memory or storage as part of the detection unit(s) 402 or transmitted in real- or near real-time. The memory or storage may be part of a system on a chip (SOC), permeant units directly coupled to the detection unit(s) 402, or removable units that are received by the detection unit(s) 402.

The data or data sets recorded or transmitted by the detection unit(s) 402 can be provided to an imager unit 404. In at least one example, the imager unit 404 may be incorporated as part of the detection unit(s) 402 or a computing device 406. In at least one embodiment, the detection unit 402 may be called a thermal detector, and the imager unit 404 may be called a thermal imager. The imager unit 404 can take the data or data sets provided by the detection unit(s) 402 for conversion into viewable images, video, or other displayable formats. In at least one embodiment, the imager unit 404 can generate two- or three-dimensional images or video from the data or data sets. These images or video may be displayed through a computing device 406, or through other devices such as, but not limited to, televisions, displays, mobile devices, tablets, phones, Personal Digital Assistants (PDAs), digital watches, smart-watches, or other devices capable of allowing viewing of information or data provided to it. In at least one example, the imager unit 404 may also overlay additional data or information, such as, but not limited to, zones, gridlines, highlights, color maps, legends, time or date information, patient information, hospital information, doctor or medical staff information, and/or other valuable information that may be received or processed by the imager unit 404. In other examples, the additional data or information may be provided by a computing device 406, a database connected to the computing device through a computer network, and/or other devices coupled to a computer network capable of transmitting data or information to the computing device 406 or the imager unit 404.

The computing device 406 can be utilized to process, analyze, network, and/or communicate information or data from the various device connected, coupled, or other interfaced with the computing device 406. In at least one embodiment, the computing device 406 is connected to the imager unit 404, a display 408, and a user interface 410. The computing device 406 can allow for the processing of images or video from the imager unit(s) 404. For example, a thermal gradient image is provided from the imager unit 404 to the computing device 406 for processing and analysis. The computing device 406 creates a set of sections or zones to be reviewed for specific temperature changes or gradients. If a section or zone is outside of the specific range of temperature changes or gradients, then an alert can be generated. In some examples, the temperature changes or gradients may need to be outside a specified range of temperature changes or gradients, and an alert can be generated if the temperature change or gradient is within the specified range. The computing device 406 may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline, or initial threshold for a temperature change or gradient during an operation. Data or data sets from the detection unit 402 may be compared to the temperature readings or data from the scan prior to the procedure or medical operation. Alerts may include sounds, notifications, emails, visual indicators, or other manner of capturing the attention of a medical professional. For example, a controller, computing device, or user interface 410 may allow for a medical professional to input or provide preferences and/or settings for the analysis of the data, data sets, images, videos, or other information provided to the computing device 406. In some examples, a controller, computing device, or user interface 410 may allow for signals, alerts, or other information to be received and acted upon to trigger additional activities or operations. In this example, a controller 410 may receive an alert, which triggers the controller to activate additional medications or requests to a pharmacy for additional medications to be available. The controller or user interface 410 can be a contrast controller to increase or decrease contrast materials to a patient or through intermediate devices to the patient. A contrast controller may also interact with or through a computing device 406 to adjust the contrast of images, video, or data received from the detection unit 402 or imager unit 404. For example, the contrast controller may adjust the detected temperature range or the scale of the temperature ranges. Further to this example, the ranges may be adjusted from a linear scale to an exponential scale or other scales as desired by the user or medical professional.

The same alert that is received by the controller 410 may also be received by an alarm 412 or similar device that can attract the attention of a medical professional or other individual that should be alerted to a change in a detected condition. In some examples, the alert may be provided to a display 408 that can allow medical professionals or other users to review the data provided and act on the information accordingly. For example, a medical professional, upon receiving the alert, may want to have a computing device 406 or controller 410 modify the settings of a pump 414 that is actively providing the fluid being perfused within the organ or tissue.

The data capture 430 can be separated into zones 432A, 432B, 432C, 432D, 432E (collectively zones 432). A heart 434 is shown as the tissue or organ to be monitored. The heart 434 can also be presented in a three-dimensional image with vertical, horizontal, and depth zones being generated. Each of these zones 432 can be monitored for specific thermal conditions or changes before, during, or after procedures or medical operations. In at least one embodiment, the thermal imaging detection system 400 is utilized to provide monitoring of perfusion of drugs utilized to slow the heart 434 during procedures or medical operations. Each zone 432 may be set individually or in select regions. For example, zones 432A, 432B, 432C, and 432D may be grouped since an artery 436 and vessels 438A, 438B, 438C, and 438D enter and/or exit through those zones 432.

In at least one example, zone 432E can be significantly different in temperature from the other zones 432. This can be an indication that perfusion is not occurring properly. When the difference or gradient change in temperature is detected with the detection unit 402, converted into an image or video with the imager unit 404, and then provided to the computing device 406. The computing device 406 can be utilized to analyze, determine, or process the data or information provided through automation, machine learning, artificial intelligence, or other computer-executed code from a storage device or computer readable media. In at least one embodiment, when the computing device 406 has analyzed, determined, or processed the data or information, it can be utilized to alert doctors or other medical professionals through an alarm 408 or display 412. A medical professional or doctor may then take actions based on the alerts, notifications, or other means of attracting the attention of the doctor or medical professional. In other examples, the thermal imaging detection system 400 may activate specified protocols or steps that the computing device 406 or other devices coupled to the thermal imaging detection system 400.

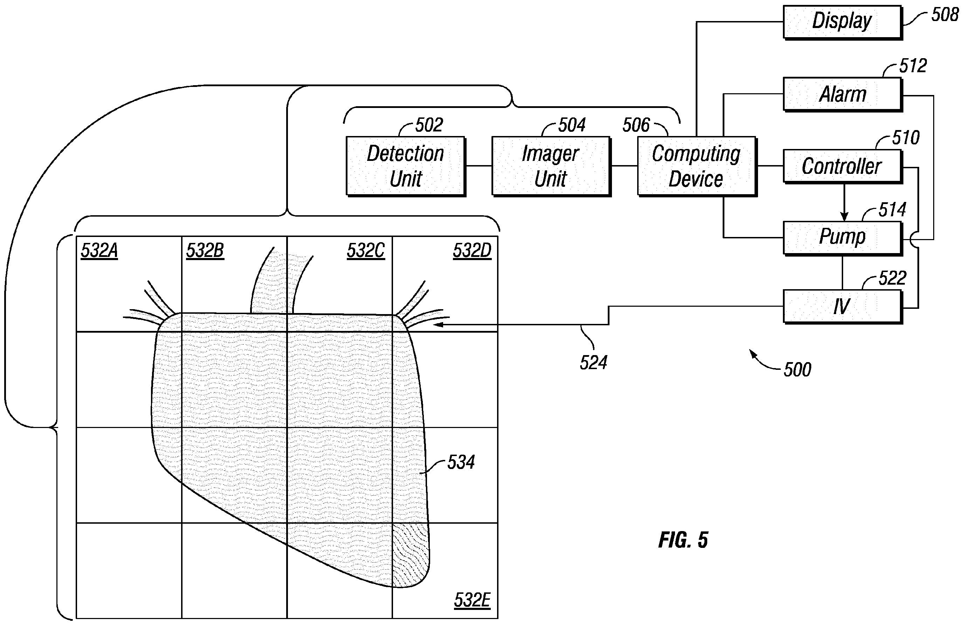

FIG. 5 is an illustration of data capture from a thermal imaging detection system 500 with an integrated feedback loop. The detection unit 502 of the thermal imaging detection system 500 can allow for a data capture 530 to occur. It should be noted, while data capture 530 is shown as an image, the data capture 530 may be processed by the imager unit 504 and/or the computing device 506 in order to show all of what is represented. In at least one embodiment, the detection unit 502 may be called a thermal detector, and the imager unit 504 may be called a thermal imager.

In at least one embodiment, the thermal imaging detection system 500 is utilized within a medical or surgical light to provide overhead thermal imaging during a procedure or related medical operation. Doctors and medical personnel often provide fluids to patients to increase or decrease temperatures for specific operations and portions of organs or tissue. For example, during procedures or related medical operations related to the heart, fluids are perfused into the heart muscle that can slow or speed up the beating of the heart muscle, and the perfusion can be monitored by the changes in a thermal gradient of the tissue. As perfusion occurs, the temperature gradient or change in the tissue can be detected and utilized to determine if proper perfusion of medications or other fluids is occurring properly. If the perfusion is not occurring properly, an alarm or other indication may be provided to individuals near the procedure or related medical operation in a manner that causes individuals to monitor the patient with greater detail.

The thermal imaging detection system 500 may utilize a detection unit 502 for capturing data regarding thermal changes to organs or tissues affected by temperature changes or gradient changes during a procedure or related medical operation. The detection unit 502 may allow for the detection of temperature changes or gradients as small as 0.001 degrees Fahrenheit, or its equivalent temperature in Celsius or Kelvin. In at least one example, detection can occur in a range from negative thirty (-30) degrees Fahrenheit, to one hundred twenty-five (125) degrees Fahrenheit, or equivalent temperatures in Celsius (-35 to 50 °C) or Kelvin. In some examples, the temperature range could extend from negative fifty (-50) degrees Fahrenheit, to one-hundred fifty (150) degrees Fahrenheit, or equivalent temperatures in Celsius or Kelvin. The data or data sets provided from the detection unit 502 may include coordinates, temperature readings, time stamps, temperature changes over time, discrete temperature, other additional data or information, comparisons, or combinations thereof. In at least one example, multiple detection unit(s) 502 may be coupled together or multiplexed for the transmission of data or data sets, to allow for multi-dimensional imaging to occur. For example, at least four detection unit(s) 502 could be coupled or fastened to four separate points that are distal from one another along with a surgical or medical light 518 utilized in procedures or other related medical operations. This can allow doctors or other medical personnel to view organs and tissues at more than one level to determine if perfusion or other monitored conditions or operations are occurring correctly or if changes to the procedure, operation, medicines, or other activities need to occur. In some examples, the data or data set generated by the detection unit(s) 502 may be stored in memory or storage as part of the detection unit(s) 502 or transmitted in real- or near real-time. The memory or storage may be part of a system on a chip (SOC), permeant units directly coupled to the detection unit(s) 502, or removable units that are received by the detection unit(s) 502.

The data or data sets recorded or transmitted by the detection unit(s) 502 can be provided to an imager unit 504. The imager unit 504 and the detection unit(s) 502 can be incorporated within a surgical or medical light 518, along with light units 516. In at least one embodiment, the detection unit 502 may be called a thermal detector, and the imager unit 504 may be called a thermal imager. In some examples, the light units 516 may have the detection unit(s) 502 integrated within in them. For example, a light unit 516 may include three light-emitting diodes or other light sources or bulbs with the detection unit 502 centrally arranged around them. In at least one embodiment, multiple detection unit(s) 502 will be positioned in multiple locations within the surgical or medical light 518 to allow for multi-dimensional data or data sets, such as but not limited to three-dimensional data or data sets to be generated. An imager unit 504 can be assigned or connected to individual detection unit(s) 502 or received a multiplexed or multiple streams of data from multiple detection units 502.

The imager unit 504 can take the data or data sets provided by the detection unit(s) 502 for conversion into viewable images, video, or other displayable formats. In at least one embodiment, the imager unit 504 can generate two- or three-dimensional images or video from the data or data sets. These images or video may be displayed through a computing device 506, or through other devices such as, but not limited to, televisions, displays, mobile devices, tablets, phones, Personal Digital Assistants (PDAs), digital watches, smart-watches, or other devices capable of allowing viewing of information or data provided to it. In at least one example, the imager unit 504 may also overlay additional data or information, such as, but not limited to, zones, gridlines, highlights, color maps, legends, time or date information, patient information, hospital information, doctor or medical staff information, and/or other valuable information that may be received or processed by the imager unit 504. In other examples, the additional data or information may be provided by a computing device 506, a database connected to the computing device through a computer network, and/or other devices coupled to a computer network capable of transmitting data or information to the computing device 506 or the imager unit 504.

The computing device 506 can be utilized to process, analyze, network, and/or communicate information or data from the various device connected, coupled, or other interfaced with the computing device 506. In at least one embodiment, the computing device 506 is connected to the imager unit 504, a display 508, and a user interface 510. The computing device 506 can allow for the processing of images or video from the imager unit(s) 504. For example, a thermal gradient image is provided from the imager unit 504 to the computing device 506, for processing and analysis. The computing device 506 creates a set of sections or zones to be reviewed for specific temperature changes or gradients. If a section or zone is outside of the specific range of temperature changes or gradients, then an alert can be generated. In some examples, the temperature changes or gradients may need to be outside a specified range of temperature changes or gradients, and an alert can be generated if the temperature change or gradient is within the specified range. The computing device 506 may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation. Data or data sets from the detection unit 502 may be compared to the temperature readings or data from the scan prior to the procedure or medical operation. Alerts may include sounds, notifications, emails, visual indicators, or other manner of capturing the attention of a medical professional. For example, a controller, computing device, or user interface 510 may allow for a medical professional to input or provide preferences and/or settings for the analysis of the data, data sets, images, videos, or other information provided to the computing device 506. In some examples, a controller, computing device, or user interface 510 may allow for signals, alerts, or other information to be received and acted upon to trigger additional activities or operations. In this example, a controller 510 may receive an alert, which triggers the controller to activate additional medications or requests to a pharmacy for additional medications to be available.

The same alert that is received by the controller 510 may also be received by an alarm 512 or similar device that can attract the attention of a medical professional or other individual that should be alerted to a change in a detected condition. In some examples, the alert may be provided to a display 508 that can allow medical professionals or other users to review the data provided and act on the information accordingly. For example, a medical professional, upon receiving the alert, may want to have a computing device 506 or controller 510 modify the settings of a pump 514 that is actively providing the fluid being perfused within the organ or tissue.

The data capture 530 can be separated into zones 532A, 532B, 532C, 532D, 532E (collectively zones 532). A heart 534 is shown as the tissue or organ to be monitored. The heart 534 can also be presented in a three-dimensional image with vertical, horizontal, and depth zones being generated. Each of these zones 532 can be monitored for specific thermal conditions or changes before, during, or after procedures or medical operations. In at least one embodiment, the thermal imaging detection system 500 is utilized to provide monitoring of perfusion of drugs utilized to slow the heart 534 during procedures or medical operations. Each zone 532 may be set individually or in select regions. For example, zones 532A, 532B, 532C, and 532D may be grouped since an artery 536, and vessels 538A, 538B, 538C, and 538D enter and/or exit through those zones 532.

In at least one example, zone 532E can be significantly different in temperature from the other zones 532. This can be an indication that perfusion is not occurring properly. When the difference or gradient change in temperature is detected with the detection unit 502, converted into an image or video with the imager unit 504, and then provided to the computing device 506. The computing device 506 can be utilized to analyze, determine, or process the data or information provided through automation, machine learning, artificial intelligence, or other computer-executed code. In at least one embodiment, when the computing device 506 has analyzed, determined, or processed the data or information, it can be utilized to alert doctors or other medical professionals through an alarm 508 or display 512. A medical professional or doctor may then take actions based on the alerts, notifications, or other means of attracting the attention of the doctor or medical professional. In other examples, the thermal imaging detection system 500 may activate specified protocols or steps that the computing device 506 or other devices coupled to the thermal imaging detection system 500.

The computing device 506, in combination with the imager unit 504 and/or detection unit 502, can process images, video, or other data with an imaging algorithm. The imaging algorithm may determine specific portions of an organ or tissue that may require additional fluids to be perfused through while also accounting for the overall condition such as temperature to be sure that a change for a specific location would not create problems for the organ or tissue overall. In other examples, the imaging algorithm may account for typical or standard values or ranges for the particular patient, organ, or tissue, as well as ambient conditions or stage of the procedure or medical operation. For example, if the thermal imaging detection system 500 is utilized with a heart procedure, the perfusion during the early stages of the procedure will likely not be as predominant, it would be in later stages of the procedure. Moreover, the imaging algorithm may be utilized before perfusion begins to allow for a baseline range to be set and utilized during the procedure. The changes over time or adjacent areas as compared with a standard or feedback from previous data, and the differences detected may be utilized by the imaging algorithm to provide alerts or feedback during a procedure or medical operation.

For example, a controller or user interface 510 may be activated to allow for control of additional medical equipment or devices within the rooms, medical suite, or surgical suite. Similarly, the alarm 508 may also be connected to additional medical equipment or devices within the rooms, medical suite, or surgical suite in a manner that would trigger actions, steps, or operations based on specific rules, alerts, or notifications. These rules may include generating alerts or notifications when a temperature passes a specified or established threshold, for example, a specified threshold may be the desired values and the established threshold may be critical values established through medical research. Other rules may include alerts or notifications, when the temperature change does or does not occur over a specified or established period of time. Yet another rule, can include, if a temperature is above or below a specified or established threshold for specified or established period of time. These temperatures or changes in temperature may occur at discrete points of time or be an average over a period of time. If any of these rules are triggered, an alert or notification may be issued based on a determination by the computing device 506 or other device utilized for analysis, or upon determination by a user or medical professional. In at least one example, if a rule is triggered the pump 514 may be turned on or shutoff, in order to provide or prevent the perfusion of a cardioplegia to a heart.

The controller or user interface 510 may allow for connection or interaction with a pump 514 or a fluid dispensing system 522. The pump 514 or fluid dispensing system 522 may trigger the delivery 524 of specific fluid(s), drugs, or combinations thereof to the heart 534. The pump 514 can interact with the fluid dispensing system 522 to allow the combination, blending, or perfusion of blood, bodily fluids, drugs, bodily fluid replacements, other fluids, or combinations thereof. As the delivery of fluids occurs, then zone 542E may change to move closer to the temperature or other condition to be monitored of zones 542A, 542B, 542C, or 542D.

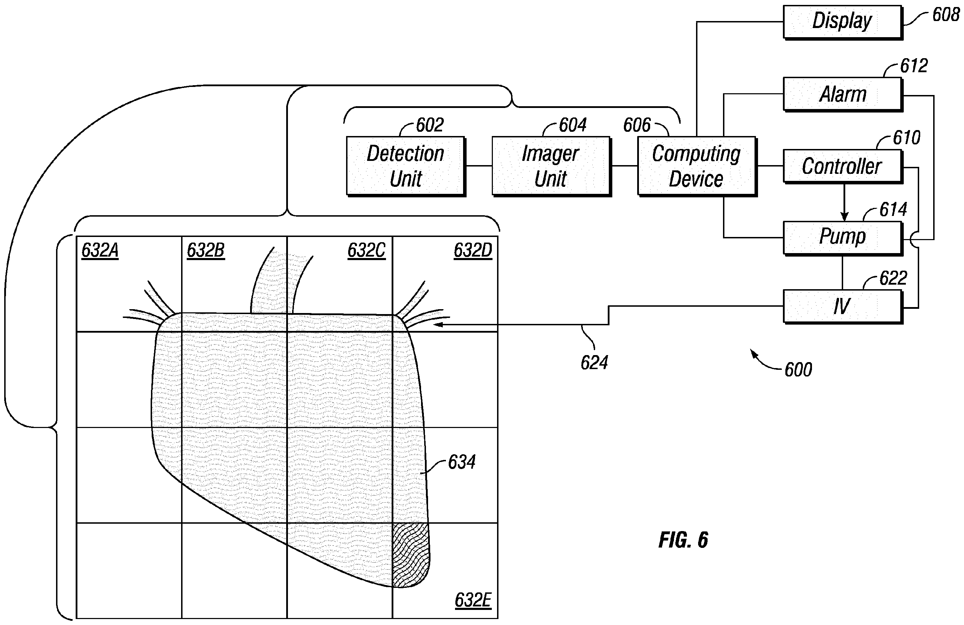

FIG. 6 is an illustration of data capture from a thermal imaging detection system with an integrated feedback loop after the execution of a feedback operation. The detection unit 602 of the thermal imaging detection system 600 can allow for a data capture 630 to occur. It should be noted, while data capture 630 is shown as an image, the data capture 630 may be processed by the imager unit 604 and/or the computing device 606 in order to show all of what is represented. In at least one embodiment, the detection unit 602 may be called a thermal detector, and the imager unit 604 may be called a thermal imager.

The thermal imaging detection system 600 can be configured to provide thermal imaging for viewing by a doctor during a procedure or related medical operation. In some examples, the thermal imaging detection system 600 can provide a feedback loop that allows for the control or recommended control of a pump 614 for delivering IV, medicines, or other fluids to a patient or specific organs or tissue 620.

In at least one example, the thermal imaging detection system 600 may be utilized within a medical or surgical light to provide overhead thermal imaging during a procedure or related medical operation. Doctors and medical personnel often provide fluids to patients to increase or decrease temperatures for specific operations and portions of organs or tissue. For example, during procedures or related medical operations related to the heart, fluids are perfused into the heart muscle that can slow or speed up the beating of the heart muscle, and the perfusion can be monitored by the changes in a thermal gradient of the tissue. A heart that slows down will have a temperature change or gradient difference from normal heart rhythms, in addition to the change in temperature or temperature gradient from the perfused fluids. As perfusion occurs, the temperature gradient or change in the tissue can be detected and utilized to determine if proper perfusion of medications or other fluids is occurring. If the perfusion is not occurring properly, an alarm or other indication may be provided to individuals near the procedure or related medical operation in a manner that causes individuals to monitor the patient with greater detail. In some examples, if perfusion is not occurring properly, a feedback loop of information or data can be analyzed to determine if the amount of fluid being perfused is proper or should be changed. While this can be done automatically, a doctor or other medical professional may trigger the change after reviewing a recommendation from the thermal imaging detection system 600.

The thermal imaging detection system 600 may utilize a detection unit 602 for capturing data regarding thermal changes to organs or tissues 620 affected by temperature changes or gradient changes during a procedure or related medical operation. The detection unit 602 may allow for the detection of temperature changes or gradients as small as 0.001 degrees Fahrenheit, or its equivalent temperature in Celsius or Kelvin. In at least one example, detection can occur in a range from negative thirty (-30) degrees Fahrenheit, to one hundred twenty-five (125) degrees Fahrenheit, or equivalent temperatures in Celsius (-35 to 50 °C) or Kelvin. In some examples, the temperature range could extend from negative fifty (-50) degrees Fahrenheit, to one-hundred fifty (150) degrees Fahrenheit, or equivalent temperatures in Celsius or Kelvin. The data or data sets provided from the detection unit 602 may include coordinates, temperature readings, time stamps, temperature changes over time, discrete temperature, other additional data or information, comparisons, or combinations thereof. In at least one example, multiple detection unit(s) 602 may be coupled together or multiplexed for the transmission of data or data sets, to allow for multi-dimensional imaging to occur. For example, at least four detection unit(s) 602 could be coupled or fastened to four separate points that are distal from one another along a surgical or medical light utilized in procedures or other related medical operations. This can allow doctors or other medical personnel to view organs and tissues at more than one level to determine if perfusion or other monitored conditions or operations are occurring correctly or if changes to the procedure, operation, medicines, or other activities need to occur. In some examples, the data or data set generated by the detection unit(s) 602 may be stored in memory or storage as part of the detection unit(s) 602 or transmitted in real- or near real-time. The memory or storage may be part of a system on a chip (SOC), permeant units directly coupled to the detection unit(s) 602, or removable units that are received by the detection unit(s) 602. In some examples, the detection unit(s) 602 or imager unit 604 may gather information regarding the ambient temperature of a room or area where the thermal image detection system 600 is being utilized.

The data or data sets recorded or transmitted by the detection unit(s) 602 can be provided to an imager unit 604. In at least one example, the imager unit 604 may be incorporated as part of the detection unit(s) 602 or a computing device 606. In at least one embodiment, the detection unit 602 may be called a thermal detector, and the imager unit 604 may be called a thermal imager. The imager unit 604 can take the data or data sets provided by the detection unit(s) 602 for conversion into viewable images, video, or other displayable formats. In at least one embodiment, the imager unit 604 can generate two- or three-dimensional images or video from the data or data sets. These images or video may be displayed through a computing device 606, or through other devices such as, but not limited to, televisions, displays, mobile devices, tablets, phones, Personal Digital Assistants (PDAs), digital watches, smart-watches, or other devices capable of allowing viewing of information or data provided to it. In at least one example, the imager unit 604 may also overlay additional data or information, such as, but not limited to, zones, gridlines, highlights, color maps, legends, time or date information, patient information, hospital information, doctor or medical staff information, and/or other valuable information that may be received or processed by the imager unit 604. In other examples, the additional data or information may be provided by a computing device 606, a database connected to the computing device through a computer network, and/or other devices coupled to a computer network capable of transmitting data or information to the computing device 606 or the imager unit 604.

The computing device 606 can be utilized to process, analyze, network, and/or communicate information or data from the various device connected, coupled, or other interfaced with the computing device 606. In at least one embodiment, the computing device 306 is connected to the imager unit 604, a display 608, and a user interface 610. The computing device 606 can allow for the processing of images or video from the imager unit(s) 604. For example, a thermal gradient image is provided from the imager unit 604 to the computing device 606 for processing and analysis. The computing device 606 creates a set of sections or zones to be reviewed for specific temperature changes or gradients. If a section or zone is outside of the specific range of temperature changes or gradients, then an alert can be generated. In some examples, the temperature changes or gradients may need to be outside a specified range of temperature changes or gradients, and an alert can be generated if the temperature change or gradient is within the specified range. The computing device 606 may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation. Data or data sets from the detection unit 602 may be compared to the temperature readings or data from the scan prior to the procedure or medical operation. Alerts may include sounds, notifications, emails, visual indicators, or other manner of capturing the attention of a medical professional. For example, a controller, computing device, or user interface 610 may allow for a medical professional to input or provide preferences and/or settings for the analysis of the data, data sets, images, videos, or other information provided to the computing device 606. The controller or user interface 610 may be utilized to control or receive signals from a fluid dispensing system 622. The fluid dispensing system 622 can be an IV or fluid control system that can utilize needleless connectors, manifolds, extension, or administration sets having check valves, roller clamps, filters, ball shutoffs, drop or drip control, or other administration tools. In some examples, a controller, computing device, or user interface 610 may allow for signals, alerts, or other information to be received and acted upon to trigger additional activities or operations. In this example, a controller 610 may receive an alert, which triggers the controller to activate additional medications or requests to a pharmacy for additional medications to be available. In yet another example, the fluid dispensing system 622 may provide additional fluids or combinations of fluids to the pump 614.

The same alert that is received by the controller 610 may also be received by an alarm 612 or similar device that can attract the attention of a medical professional or other individual that should be alerted to a change in a detected condition. In some examples, the alert may be provided to a display 608 that can allow medical professionals or other users to review the data provided and act on the information accordingly. For example, a medical professional, upon receiving the alert, may want to have a computing device 606 or controller 610 modify the settings of a pump 614 that is actively providing the fluid being perfused within the organ or tissue. One example of a pump 614 can be a Quest Medical^{®} MPS3 Myocardial Protection System or similar systems, that can include monitoring of the temperature of the fluid being provided to the patient, multiple pumping units within the pump 614 to allow for fluid and blood to be pumped through the pump 614 in at least one example. In some examples, the pump 614 may also include information regarding the cardiac condition of a patient, such as the cardioplegia ratio, potassium, ECG, blood pressure, flow rates, and other information regarding the patient or the specific organs or tissues 620.

In at least one example, temperature gradients or changes are utilized to ascertain cardioplegia (CPG) distribution for effective cardioprotection or protection of the myocardium. The determination of the cardioplegia distribution allows for intra-operative decisions on cardioplegia delivery based on routes of delivery such as but not limited to antegrade, retro or simulgrade, flow and pressure rates or time of delivery to achieve optimal distribution to ensure optimal protection of the heart. This allows users or medical professionals to customize cardioplegia delivery for specific underlying diseases, conditions, or patient anatomies. Users or medical professionals can utilize the presentation of images, videos, data or information, and/or the processing or analysis to provide decision assistance for the various parameters, conditions, or rules to provide to a fluid dispensing system or pump through an open loop system. For example, a representation through a temperature or heat map can allow for surface or volume distribution of a cardioplegia through a heart and allow a user or medical processional to make adjustments to the delivery, delivery method, and/or delivery positioning to provide optimal delivery. Alternatively, the fluid dispensing system or pump may be controlled or auto regulated in a closed loop system based on the various parameters, conditions, or rules. For example, the flow rate of a cardioplegia can be controlled or auto regulated in the closed loop system.

In at least one embodiment, the computing device 606 is connected with the controller or user interface 610, the alarm 612, and the pump 614. The alarm 612 may also be coupled to the pump 614 to allow it to trigger alarms, alerts, notifications, or otherwise attracting the attention of a medical professional or other user based on information or signals from the computing device 606 or the pump 614. The pump 614 can receive fluids from a fluid dispensing system 622 that can be perfused within a blood supply pumped through the pump 614 or directly to the tissue or organ 620. The pump 614 may adjust the pumping operations, perfusion, or other operations based on signals from the computing device 606 or other instructions received from a medical professional or other user.

In at least one example, the computing device 606 can be coupled directly or indirectly to the detection unit 602 and/or the image unit 604, and the computing device 606 provides the image, video, data or information to a display 608 connected directly to the computing device 606. In some examples, the detection unit 602 and/or the image unit 604 can be coupled to the computing device 606 that is coupled to a user interface 610, which can be, but is not limited to, an operating room or surgical suite monitor system. Yet, in other examples, the computing device 606 can be coupled directly or indirectly to the detection unit 602 and/or the image unit 604, and the computing device 606 provides the image, video, data or information to a display 608 connected through a network or wireless connection to the computing device 606. Similarly, the image, video, data or information provided from the computing device 606, via the detection unit 602 and/or the image unit 604 may be provided to the fluid dispensing system 622 and/or the pump 614 either of which may have a display or monitor to provide the visual representations.

The computing device 606, in at least one example, can be configured to record or log images, video, or representations of data from the detection unit 602 and/or imager unit 604. Similarly, the images, video, or representations of data from the detection unit 602, and/or imager unit 604 may be played back by the computing device 606 to other devices coupled to the computing devices 606. Data logging of images, video, data or information from the detection unit 602 and/or imager unit 604, may allow for data and/or information to be gathered separated from personal information of a patient for analysis and further improvement of processing, algorithms, and/or analysis of image, video, data or information. In at least one embodiment, a user or medical professional can select data points, images, or video to determine discrete, average, maximum, minimum, and/or range of temperature at discrete moments in time or over a specified timer period. The computing device 606 may also allow for alerts or notification to be generated when temperatures are over or under a discreate or average temperature, over or under a specified temperature of a period of time or exceeded or not exceeded a specified temperature at discrete times, or over a period of time.

In at least one embodiment, the computing device 606 can be utilized for a control system that may comprise the pump 614, fluid dispensing system 622, alarm 612, and/or controller or user interface 610. The control system can allow for the clinical intervention if a patient's condition does not comply with normal ranges. The clinical intervention can be activated by a doctor or medical professional, based on detected temperatures or temperature changes. The control system can provide a feedback loop of information to determine if changes made by the control system or by a user are having the desired effects or if a different intervention may be desired.

The data capture 630 can be separated into zones 632A, 632B, 632C, 632D, 632E (collectively zones 632). A heart 634 is shown as the tissue or organ to be monitored. The heart 634 can also be presented in a three-dimensional image with vertical, horizontal, and depth zones being generated. Each of these zones 632 can be monitored for specific thermal conditions or changes before, during, or after procedures or medical operations. In at least one embodiment, the thermal imaging detection system 600 is utilized to provide monitoring of perfusion of drugs utilized to slow the heart 634 during procedures or medical operations. Each zone 632 may be set individually or in select regions. For example, zones 632A, 632B, 632C, and 632D may be grouped since an artery 636, and vessels 638A, 638B, 638C, and 638D enter and/or exit through those zones 632.

In at least one example, zone 632E can be significantly different in temperature from the other zones 632. This can be an indication that perfusion is not occurring properly. When the difference or gradient change in temperature is detected with the detection unit 602, converted into an image or video with the imager unit 604, and then provided to the computing device 606. The computing device 606 can be utilized to analyze, determine or process the data or information provided through automation, machine learning, artificial intelligence, or other computer-executed code. In at least one embodiment, when the computing device 606 has analyzed, determined, or processed the data or information, it can be utilized to alert doctors or other medical professionals through an alarm 608 or display 612. A medical professional or doctor may then take actions based on the alerts, notifications, or other means of attracting the attention of the doctor or medical professional. In other examples, the thermal imaging detection system 600 may activate specified protocols or steps that the computing device 606 or other devices coupled to the thermal imaging detection system 600.

For example, a controller or user interface 610 may be activated to allow for control of additional medical equipment or devices within the rooms, medical suite, or surgical suite. Similarly, the alarm 608 may also be connected to additional medical equipment or devices within the rooms, medical suite, or surgical suite in a manner that would trigger actions, steps, or operations based on specific rules, alerts, or notifications. These rules may include generating alerts or notifications when a temperature passes a specified or established threshold, for example, a specified threshold may be the desired values and the established threshold may be critical values established through medical research. Other rules may include alerts or notifications, when the temperature change does or does not occur over a specified or established period of time. Yet another rule, can include, if a temperature is above or below a specified or established threshold for specified or established period of time. These temperatures or changes in temperature may occur at discrete points of time or be an average over a period of time. If any of these rules are triggered, an alert or notification may be issued based on a determination by the computing device 306 or other device utilized for analysis, or upon determination by a user or medical professional. In at least one example, if a rule is triggered the pump 614 may be turned on or shutoff, in order to provide or prevent the perfusion of a cardioplegia to a heart.

The controller or user interface 610 may allow for connection or interaction with a pump 614 or a fluid dispensing system 622. The pump 614 or fluid dispensing system 622 may trigger the delivery 624 of specific fluid(s), drugs, or combinations thereof to the heart 634. The pump 614 can interact with the fluid dispensing system 622 to allow the combination, blending, or perfusion of blood, bodily fluids, drugs, bodily fluid replacements, other fluids, or combinations thereof. As the delivery of fluids occurs, then zone 642E may change to move closer to the temperature or other conditions to be monitored of zones 642A, 642B, 642C, or 642D. After complete perfusion or delivery 624 of fluids, the heart 634 may normalize across all zones 642. These changes can be detected with the detection unit 602 and provided to the computing device 606 through the imager unit 604. In at least one embodiment, the detection unit 602 may be called a thermal detector, and the imager unit 604 may be called a thermal imager.

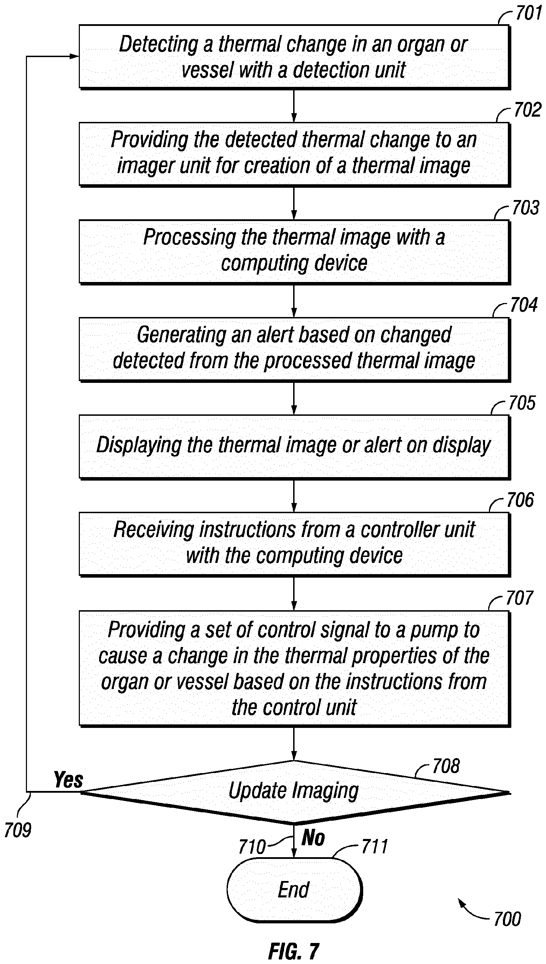

FIG. 7 is an illustration of an operational flow for thermal imaging detection 700. In step 701, a detection unit can be utilized for detecting a thermal change in an organ or vessel. In at least one example, the detection unit may be incorporated into a surgical or medical light for use in an operating room or in medical facilities. The detection unit can capture data regarding thermal changes to organs or tissues as small as 0.001 degrees Fahrenheit or its equivalent temperature in Celsius or Kelvin. In at least one example, detection can occur in a range from negative thirty (-30) degrees Fahrenheit, to one hundred twenty-five (125) degrees Fahrenheit, or equivalent temperatures in Celsius (-35 to 50 °C) or Kelvin. In some examples, the temperature range could extend from negative fifty (-50) degrees Fahrenheit, to one-hundred fifty (150) degrees Fahrenheit, or equivalent temperatures in Celsius or Kelvin. These temperature changes or gradient changes can occur prior to, during, or after a procedure or related medical operation.

The data or data sets captured, recorded, or transmitted by the detection unit may include coordinates, temperature readings, time stamps, temperature changes over time, discrete temperature, other additional data or information, comparisons, or combinations thereof. In at least one example, multiple detection unit(s) may be coupled together or multiplexed for the transmission of data or data sets to allow for multi-dimensional imaging to occur. For example, at least four detection unit(s) could be coupled or fastened to four separate points that are distal from one another along a surgical or medical light utilized in procedures or other related medical operations. Multi-dimensional imaging can allow doctors or other medical personnel to view organs and tissues at more than one level of depth or penetration to determine if perfusion or other monitored conditions or operations are occurring correctly or if changes to the procedure, operation, medicines, or other activities need to occur.

In some examples, the data or data set generated by the detection unit(s) may be stored in memory or storage as part of the detection unit(s) or transmitted in real- or near real-time. The memory or storage may be part of a system on a chip (SOC), permeant units directly coupled to the detection unit(s), or removable units that are received by the detection unit(s).

In step 702, the detected thermal change can be provided to an imager unit for the creation of a thermal image. In at least one embodiment, the detection unit is utilized for providing the detected thermal change to the imager unit. In some examples, the data or data sets recorded or transmitted by the detection unit(s) can be provided to an imager unit through a wired or wireless connection or through a computer network. Additionally, the detection unit(s) or imager unit may gather information regarding the ambient temperature of a room or area where the thermal image detection system is being utilized.

It should be noted that, while data capture is shown as an image, the data capture may be processed by the imager unit and/or the computing device in order to provide a complete representation. In at least one example, the imager unit may be incorporated as part of the detection unit(s) or a computing device. The imager unit can take the data or data sets provided by the detection unit(s) for conversion into viewable images, video, or other displayable formats. In at least one embodiment, the imager unit can generate two- or three-dimensional images or video from the data or data sets. These images or video may be displayed through a computing device or through other devices such as, but not limited to, televisions, displays, mobile devices, tablets, phones, Personal Digital Assistants (PDAs), digital watches, smart-watches, or other devices capable of allowing viewing of information or data provided to it. In at least one example, the imager unit may also overlay additional data or information, such as, but not limited to, zones, gridlines, highlights, color maps, legends, time or date information, patient information, hospital information, doctor or medical staff information, and/or other valuable information that may be received or processed by the imager unit. In other examples, the additional data or information may be provided by a computing device, a database connected to the computing device through a computer network, and/or other devices coupled to a computer network capable of transmitting data or information to the computing device or the imager unit.

In step 703, processing the thermal image can be performed with a computing device. The processing may include analysis, comparison, deconstruction, reconstruction, transformation, conversion, other image or video processing operations or steps, or combinations thereof. The computing device can be utilized to process, analyze, network, and/or communicate information or data from the various device connected, coupled, or other interfaced with the computing device. In at least one embodiment, the computing device is connected to the imager unit, a display, and a user interface. In at least one example, the computing device creates a set of sections or zones to be reviewed for specific temperature changes or gradients. If a section or zone is outside of the specific range of temperature changes or gradients, then an alert can be generated. In some examples, the temperature changes or gradients may need to be outside a specified range of temperature changes or gradients, and an alert can be generated if the temperature change or gradient is within the specified range.

Data or data sets from the detection unit that have been processed or analyzed may be compared to the temperature readings or data from the scan prior to the procedure or medical operation. Similarly, in some examples, the computing device may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation. For example, a controller, computing device, or user interface may allow for a medical professional to input or provide preferences and/or settings for the analysis of the data, data sets, images, videos, or other information provided to the computing device.

The data capture can be separated into zones. For example, if the data capture includes a heart or other organ or tissue, it can be presented to a user or medical professional in a three-dimensional image with vertical, horizontal, and depth zones being generated. Each of these zones can be monitored for specific thermal conditions or changes before, during, or after procedures or medical operations. In at least one embodiment, the thermal imaging detection system is utilized to provide monitoring of perfusion of drugs utilized to slow the heart during procedures or medical operations. Each zone may be set individually or in select regions for specific conditions, ranges, or other options for monitoring. For example, zones may be grouped for a heart as the areas where an artery or vessels enter and/or exit.

In at least one example, a single zone may have a significantly different temperature from adjacent zones. This can be an indication that perfusion is not occurring properly in that particular zone of the organ or tissue being monitored. The computing device can be utilized to analyze, determine, or process the data or information provided through automation, machine learning, artificial intelligence, or other computer-executed code. In at least one embodiment, when the computing device has analyzed, determined, or processed the data or information for each zone, region, or complete image or video, the computing device can be utilized to alert doctors or other medical professionals through an alarm or display.

In step 704, generating an alert based on a change detected from the processed thermal image. In at least one embodiment, the alert is generated by the computing device. In some examples, the alert may be generated based on a change detected in a comparison of multiple processed thermal images or videos. A medical professional or doctor may then take actions based on the alerts, notifications, or other means of attracting the attention of the doctor or medical professional. Alerts may include sounds, notifications, emails, visual indicators, or other manners of capturing the attention of a medical professional. In other examples, the thermal imaging detection system may activate specified protocols or steps that the computing device or other devices coupled to the thermal imaging detection system may execute.

Doctors and medical personnel often provide fluids to patients to increase or decrease temperatures for specific operations and portions of organs or tissue. For example, during procedures or related medical operations related to the heart, fluids are perfused into the heart muscle that can slow or speed up the beating of the heart muscle, and the perfusion can be monitored by the changes in a thermal gradient of the tissue. A heart that slows down will have a temperature change or gradient difference from normal heart rhythms, in addition to the change in temperature or temperature gradient from the perfused fluids. As perfusion occurs, the temperature gradient or change in the tissue can be detected and utilized to determine if proper perfusion of medications or other fluids is occurring. If the perfusion is not occurring properly, an alarm or other indication may be provided to individuals near the procedure or related medical operation in a manner that causes individuals to monitor the patient with greater detail. In some examples, if perfusion is not occurring properly, a feedback loop of information or data can be analyzed to determine if the amount of fluid being perfused is proper or should be changed. While this can be done automatically, a doctor or other medical professional may trigger the change after reviewing a recommendation from the thermal imaging detection system.

For example, a controller, computing device, or user interface may allow for a medical professional to input or provide preferences and/or settings for the analysis of the data, data sets, images, videos, or other information provided to the computing device. In some examples, a controller, computing device, or user interface may allow for signals, alerts, or other information to be received and acted upon to trigger additional activities or operations. In this example, a controller may receive an alert, which triggers the controller to activate additional medications or requests to a pharmacy for additional medications to be available.

The same alert that is received by the controller may also be received by an alarm or similar device that can attract the attention of a medical professional or other individual that should be alerted to a change in a detected condition. In some examples, the alert may be provided to a display that can allow medical professionals or other users to review the data provided and act on the information accordingly. For example, a medical professional, upon receiving the alert, may want to have a computing device or controller modify the settings of a pump that is actively providing the fluid being perfused within the organ or tissue. One example of a pump can be a Quest Medical^{®} MPS3 Myocardial Protection System or similar systems, which can include monitoring of the temperature of the fluid being provided to the patient, multiple pumping units within the pump to allow for fluid and blood to be pumped through the pump in at least one example. In some examples, the pump may also include information regarding the cardiac condition of a patient, such as the cardioplegia ratio, potassium, ECG, blood pressure, flow rates, and other information regarding the patient or the specific organs or tissues.

In at least one example, temperature gradients or changes are utilized to ascertain cardioplegia (CPG) distribution for effective cardioprotection or protection of the myocardium. The determination of the cardioplegia distribution allows for intra-operative decisions on cardioplegia delivery based on routes of delivery such as but not limited to antegrade, retro or simulgrade, flow and pressure rates or time of delivery to achieve optimal distribution to ensure optimal protection of the heart. This allows users or medical professionals to customize cardioplegia delivery for specific underlying diseases, conditions, or patient anatomies. Users or medical professionals can utilize the presentation of images, videos, data or information, and/or the processing or analysis to provide decision assistance for the various parameters, conditions, or rules to provide to a fluid dispensing system or pump through an open loop system. For example, a representation through a temperature or heat map can allow for surface or volume distribution of a cardioplegia through a heart and allow a user or medical processional to make adjustments to the delivery, delivery method, and/or delivery positioning to provide optimal delivery. Alternatively, the fluid dispensing system or pump may be controlled or auto regulated in a closed loop system based on the various parameters, conditions, or rules. For example, the flow rate of a cardioplegia can be controlled or auto regulated in the closed loop system.

Step 705 depicts the thermal image or alert on a display. In at least one embodiment, the displayed thermal image may be the processed thermal image. In some examples, the display may be a computer monitor, tv, tablet, phone, or other devices capable of displaying an image, video, or information.

In at least one embodiment, when the computing device has analyzed, determined, or processed the data or information, it can be utilized to alert doctors or other medical professionals through an alarm or display. In some examples, the alert may be provided to a display that can allow medical professionals or other users to review the data provided and act on the information accordingly. For example, a medical professional, upon receiving the alert, may want to have a computing device or controller modify the settings of a pump that is actively providing the fluid being perfused within the organ or tissue. In other examples, the thermal imaging detection system may activate specified protocols or steps that the computing device or other devices coupled to the thermal imaging detection system. Similarly, the alarm may also be connected to additional medical equipment or devices within the rooms, medical suite, or surgical suite in a manner that would trigger actions, steps, or operations based on specific rules, alerts, or notifications. These rules may include generating alerts or notifications when a temperature passes a specified or established threshold, for example, a specified threshold may be the desired values and the established threshold may be critical values established through medical research. Other rules may include alerts or notifications, when the temperature change does or does not occur over a specified or established period of time. Yet another rule, can include, if a temperature is above or below a specified or established threshold for specified or established period of time. These temperatures or changes in temperature may occur at discrete points of time or be an average over a period of time. If any of these rules are triggered, an alert or notification may be issued based on a determination by the computing device or other device utilized for analysis, or upon determination by a user or medical professional. In at least one example, if a rule is triggered the pump may be turned on or shutoff, in order to provide or prevent the perfusion of a cardioplegia to a heart.

Step 706 shows the receiving of instructions from a controller unit with the computing device. In at least one example, the controller unit may be a user interface or other device capable of receiving interactions or inputs from a user, such as but not limited to, a doctor, nurse, or other medical professional. For example, a controller, computing device, or user interface may allow for a medical professional to input or provide preferences and/or settings for the analysis of the data, data sets, images, videos, or other information provided to the computing device. The controller or user interface may be utilized to control or receive signals from a fluid dispensing system. The fluid dispensing system can be an IV or fluid control system that can utilize needleless connectors, manifolds, extension, or administration sets having check valves, roller clamps, filters, ball shutoffs, drop or drip control, or other administration tools. In some examples, a controller, computing device, or user interface may allow for signals, alerts, or other information to be received and acted upon to trigger additional activities or operations. In this example, a controller may receive an alert, which triggers the controller to activate additional medications or requests to a pharmacy for additional medications to be available. In yet another example, the fluid dispensing system may provide additional fluids or combinations of fluids to the pump.

The same alert that is received by the controller may also be received by an alarm or similar device that can attract the attention of a medical professional or other individual that should be alerted to a change in a detected condition. In some examples, the alert may be provided to a display that can allow medical professionals or other users to review the data provided and act on the information accordingly. For example, a medical professional, upon receiving the alert, may want to have a computing device or controller modify the settings of a pump that is actively providing the fluid being perfused within the organ or tissue. In at least one embodiment, the computing device can be utilized for a control system that may comprise the pump, fluid dispensing system, alarm, and/or controller or user interface. For example, a controller or user interface may be activated to allow for control of additional medical equipment or devices within the rooms, medical suite, or surgical suite. Similarly, the alarm may also be connected to additional medical equipment or devices within the rooms, medical suite, or surgical suite in a manner that would trigger actions, steps, or operations based on specific rules, alerts, or notifications. These rules may include generating alerts or notifications when a temperature passes a specified or established threshold, for example, a specified threshold may be the desired values and the established threshold may be critical values established through medical research. Other rules may include alerts or notifications, when the temperature change does or does not occur over a specified or established period of time. Yet another rule, can include, if a temperature is above or below a specified or established threshold for specified or established period of time. These temperatures or changes in temperature may occur at discrete points of time or be an average over a period of time. If any of these rules are triggered, an alert or notification may be issued based on a determination by the computing device or other device utilized for analysis, or upon determination by a user or medical professional. In at least one example, if a rule is triggered the pump may be turned on or shutoff, in order to provide or prevent the perfusion of a cardioplegia to a heart.

The controller or user interface can also be a contrast controller to increase or decrease contrast materials to a patient or through intermediate devices to the patient. A contrast controller may also interact with or through a computing device to adjust the contrast of images, video, or data received from the detection unit or imager unit. For example, the contrast controller may adjust the detected temperature range or the scale of the temperature ranges. Further to this example, the ranges may be adjusted from a linear scale to an exponential scale or other scales as desired by the user or medical professional.

Step 707 is providing a set of control signals to a pump to cause a change in the thermal properties of the organ or vessel based on the instructions from the control unit. In at least one example, the fluid dispensing system may provide additional fluids or combinations of fluids to the pump and receive the set of control signals along with the pump. The fluid dispensing system can be an IV or fluid control system that can utilize needleless connectors, manifolds, extension, or administration sets having check valves, roller clamps, filters, ball shutoffs, drop or drip control, or other administration tools. The pump can receive fluids from a fluid dispensing system that can be perfused within a blood supply pumped through the pump or directly to the tissue or organ. The pump may adjust the pumping operations, perfusion, or other operations-based onset of control signals from the computing device or other instructions received from a medical professional or other user. The pump can interact with the fluid dispensing system to allow the combination, blending, or perfusion of blood, bodily fluids, drugs, bodily fluid replacements, other fluids, or combinations thereof. As the delivery of fluids occurs, the particular zone being monitored may change to move closer to the desired temperature or other conditions to be monitored and allow for the temperature or other condition to normalize across all zones.

In at least one embodiment, the computing device can be utilized for a control system that may comprise the pump, fluid dispensing system, alarm, and/or controller or user interface. The control system can allow for the clinical intervention if a patient's condition does not comply with normal ranges. The clinical intervention can be activated by a doctor or medical professional, based on detected temperatures or temperature changes. The control system can provide a feedback loop of information to determine if changes made by the control system or by a user are having the desired effects or if a different intervention may be desired.

In step 708, an updating of the imaging can occur based on automatic processing instructions or through an interaction with a user. If the user selects, or an update is done automatically 709, the method can return to step 701 or any intervening step. Alternatively, if the method is complete without out additional updates 710, the user can select to end 711.

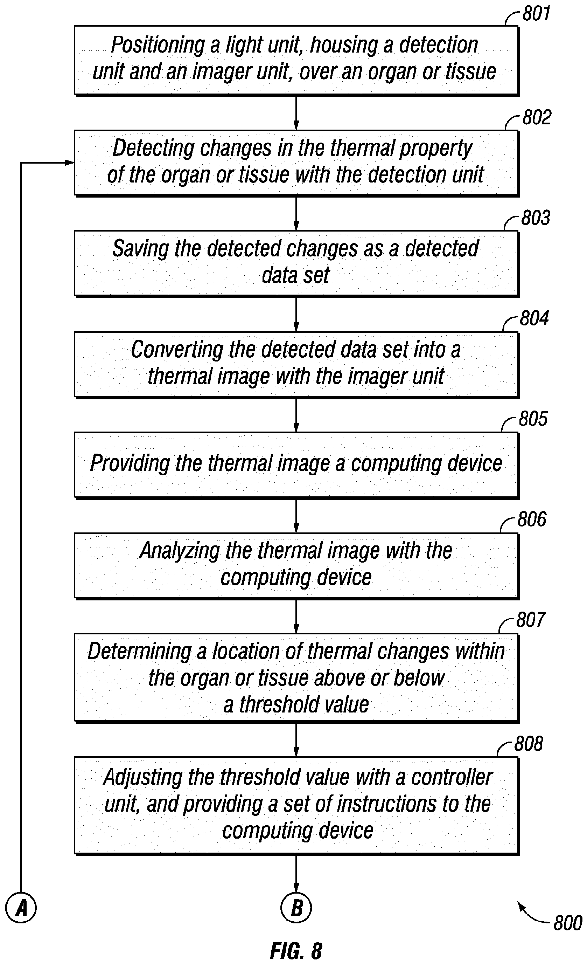

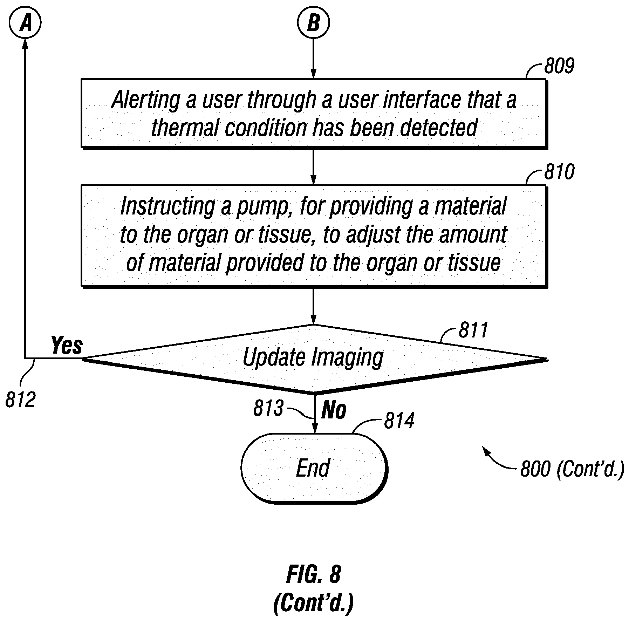

FIG. 8 is an illustration of an operational flow 800 for thermal imaging detection from a surgical or medical light. In step 801, a user may position a light unit above or near a patient. In at least one example, the light unit may be within an enclosure that also houses a detection unit and an imager unit over an organ or tissue of a patient. In at least one embodiment, the light unit may include multiple detection units and/or multiple imager units. The multiple detection unit(s) can be positioned in multiple locations within the surgical or medical light to allow for multi-dimensional data or data sets, such as but not limited to, three-dimensional data or data sets to be generated.

In step 802, the detection unit can be utilized for detecting changes in the thermal property of the organ or tissue. In at least one example, the detection unit may be incorporated into a surgical or medical light for use in an operating room or in medical facilities. The detection unit can capture data regarding thermal changes to organs or tissues as small as 0.001 degrees Fahrenheit or its equivalent temperature in Celsius or Kelvin. In at least one example, detection can occur in a range from negative thirty (-30) degrees Fahrenheit, to one hundred twenty-five (125) degrees Fahrenheit, or equivalent temperatures in Celsius (-35 to 50 °C) or Kelvin. In some examples, the temperature range could extend from negative fifty (-50) degrees Fahrenheit, to one-hundred fifty (150) degrees Fahrenheit, or equivalent temperatures in Celsius or Kelvin. These temperature changes or gradient changes can occur prior to, during, or after a procedure or related medical operation.

The data or data sets captured, recorded, or transmitted by the detection unit may include coordinates, temperature readings, time stamps, temperature changes over time, discrete temperature, other additional data or information, comparisons, or combinations thereof. In at least one example, multiple detection unit(s) may be coupled together or multiplexed for the transmission of data or data sets to allow for multi-dimensional imaging to occur. For example, at least four detection unit(s) could be coupled or fastened to four separate points that are distal from one another along a surgical or medical light utilized in procedures or other related medical operations. Multi-dimensional imaging can allow doctors or other medical personnel to view organs and tissues at more than one level of depth or penetration to determine if perfusion or other monitored conditions or operations are occurring correctly or if changes to the procedure, operation, medicines, or other activities need to occur.

In some examples, the data or data set generated by the detection unit(s) may be stored in memory or storage as part of the detection unit(s) or transmitted in real- or near real-time. The memory or storage may be part of a system on a chip (SOC), permeant units directly coupled to the detection unit(s), or removable units that are received by the detection unit(s).

Step 803 depicts saving the detected changes as a detected data set. In at least one example, the detected data set can include image or video data, time and date information, and/or positioning coordinates. Data or data sets from the detection unit may be compared to the temperature readings or data from a scan prior to the procedure or medical operation that are saved in the same storage location or device. In at least one example, the data or data sets recorded or transmitted by the detection unit(s) can be provided to an imager unit directly or through an intermediary storage device. In other examples, the additional data or information may be provided by a computing device, a database connected to the computing device through a computer network, and/or other devices coupled to a computer network capable of transmitting data or information to the computing device or the imager unit.

The computing device may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation.

In step 804, an imager unit can be utilized for converting the detected data set into a thermal image. In at least one example, the imager unit can also produce video as well. In at least one embodiment, the detection unit is utilized for providing the detected thermal change to the imager unit. In some examples, the data or data sets recorded or transmitted by the detection unit(s) can be provided to an imager unit through a wired or wireless connection or through a computer network. An imager unit can be assigned or connected to an individual detection unit(s) or received a multiplexed or multiple streams of data from multiple detection units. Additionally, the detection unit(s) or imager unit may gather information regarding the ambient temperature of a room or area where the thermal image detection system is being utilized.

It should be noted that while data capture is shown as an image, the data capture may be processed by the imager unit and/or the computing device in order to provide a complete representation. In at least one example, the imager unit may be incorporated as part of the detection unit(s) or a computing device. The imager unit can take the data or data sets provided by the detection unit(s) for conversion into viewable images, video, or other displayable formats. In at least one embodiment, the imager unit can generate two- or three-dimensional images or video from the data or data sets. These images or video may be displayed through a computing device or through other devices such as, but not limited to, televisions, displays, mobile devices, tablets, phones, Personal Digital Assistants (PDAs), digital watches, smart-watches, or other devices capable of allowing viewing of information or data provided to it. In at least one example, the imager unit may also overlay additional data or information, such as, but not limited to, zones, gridlines, highlights, color maps, legends, time or date information, patient information, hospital information, doctor, or medical staff information, and/or other valuable information that may be received or processed by the imager unit. In other examples, the additional data or information may be provided by a computing device, a database connected to the computing device through a computer network, and/or other devices coupled to a computer network capable of transmitting data or information to the computing device or the imager unit.

In step 805, the thermal image may be provided to a computing device. The computing device may receive the images, data, video, or other information directly or indirectly over a computer network or through a storage device such as but not limited to a hard drive, service, distributed storage, flash memory, or other storage device or combinations thereof. The computing device can be utilized to process, analyze, network, and/or communicate information or data from the various device connected, coupled, or other interfaced with the computing device.

Step 806 is analyzing the thermal image with the computing device. In at least one embodiment, the computing device creates a set of sections or zones to be reviewed for specific temperature changes or gradients. If a section or zone is outside of the specific range of temperature changes or gradients, then an alert can be generated by the computing device. Alerts may include sounds, notifications, emails, visual indicators, or other manner of capturing the attention of a medical professional.

In some examples, the temperature changes or gradients may need to be outside a specified range of temperature changes or gradients, and an alert can be generated if the temperature change or gradient is within the specified range. The computing device may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation.

Step 807 is determining a location of thermal changes within the organ or tissue above or below a threshold value. In at least one embodiment, the location of the thermal changes may be found through zone or coordinate analysis and/or comparison to previous thermal images or data.

The data capture can be separated into zones. For example, if the data capture includes a heart or other organ or tissue, it can be presented to a user or medical professional in a three-dimensional image with vertical, horizontal, and depth zones being generated. Each of these zones can be monitored for specific thermal conditions or changes before, during, or after procedures or medical operations. In at least one embodiment, the thermal imaging detection system is utilized to provide monitoring of perfusion of drugs utilized to slow the heart during procedures or medical operations. Each zone may be set individually or in select regions for specific conditions, ranges, or other options for monitoring. For example, zones may be grouped for a heart as the areas where an artery or vessels enter and/or exit.

In at least one example, a single zone may have a significantly different temperature from adjacent zones. This can be an indication that perfusion is not occurring properly in that particular zone of the organ or tissue being monitored. The computing device can be utilized to analyze, determine, or process the data or information provided through automation, machine learning, artificial intelligence, or other computer-executed code. In at least one embodiment, when the computing device has analyzed, determined, or processed the data or information for each zone, region, or complete image or video, the computing device can be utilized to alert doctors or other medical professionals through an alarm or display.

In step 808, a controller unit can be utilized for adjusting the threshold value and providing a set of instructions to the computing device. In at least one embodiment, the controller unit can include a user interface. Doctors and medical personnel often provide fluids to patients to increase or decrease temperatures for specific operations and portions of organs or tissue. For example, during procedures or related medical operations related to the heart, fluids are perfused into the heart muscle that can slow or speed up the beating of the heart muscle, and the perfusion can be monitored by the changes in a thermal gradient of the tissue. A heart that slows down will have a temperature change or gradient difference from normal heart rhythms, in addition to the change in temperature or temperature gradient from the perfused fluids. As perfusion occurs, the temperature gradient or change in the tissue can be detected and utilized to determine if proper perfusion of medications or other fluids is occurring. If the perfusion is not occurring properly, an alarm or other indication may be provided to individuals near the procedure or related medical operation in a manner that causes individuals to monitor the patient with greater detail. In some examples, if perfusion is not occurring properly, a feedback loop of information or data can be analyzed to determine if the amount of fluid being perfused is proper or should be changed. While this can be done automatically, a doctor or other medical professional may trigger the change after reviewing a recommendation from the thermal imaging detection system.

In at least one example, the controller unit may be a user interface or other device capable of receiving interactions or inputs from a user, such as but not limited to, a doctor, nurse, or other medical professional. For example, a controller, computing device, or user interface may allow for a medical professional to input or provide preferences and/or settings for the analysis of the data, data sets, images, videos, or other information provided to the computing device. The controller or user interface may be utilized to control or receive signals from a fluid dispensing system. The fluid dispensing system can be an IV or fluid control system that can utilize needleless connectors, manifolds, extension, or administration sets having check valves, roller clamps, filters, ball shutoffs, drop or drip control, or other administration tools. In some examples, a controller, computing device, or user interface may allow for signals, alerts, or other information to be received and acted upon to trigger additional activities or operations. In this example, a controller may receive an alert, which triggers the controller to activate additional medications or requests to a pharmacy for additional medications to be available. In yet another example, the fluid dispensing system may provide additional fluids or combinations of fluids to the pump.

The same alert that is received by the controller may also be received by an alarm or similar device that can attract the attention of a medical professional or other individual that should be alerted to a change in a detected condition. In some examples, the alert may be provided to a display that can allow medical professionals or other users to review the data provided and act on the information accordingly. For example, a medical professional, upon receiving the alert, may want to have a computing device or controller modify the settings of a pump that is actively providing the fluid being perfused within the organ or tissue. In at least one embodiment, the computing device can be utilized for a control system that may comprise the pump, fluid dispensing system, alarm, and/or controller or user interface. For example, a controller or user interface may be activated to allow for control of additional medical equipment or devices within the rooms, medical suite, or surgical suite. Similarly, the alarm may also be connected to additional medical equipment or devices within the rooms, medical suite, or surgical suite in a manner that would trigger actions, steps, or operations based on specific rules, alerts, or notifications. These rules may include generating alerts or notifications when a temperature passes a specified or established threshold, for example, a specified threshold may be the desired values and the established threshold may be critical values established through medical research. Other rules may include alerts or notifications, when the temperature change does or does not occur over a specified or established period of time. Yet another rule, can include, if a temperature is above or below a specified or established threshold for specified or established period of time. These temperatures or changes in temperature may occur at discrete points of time or be an average over a period of time. If any of these rules are triggered, an alert or notification may be issued based on a determination by the computing device or other device utilized for analysis, or upon determination by a user or medical professional. In at least one example, if a rule is triggered the pump may be turned on or shutoff, in order to provide or prevent the perfusion of a cardioplegia to a heart.

The controller or user interface can also be a contrast controller to increase or decrease contrast materials to a patient or through intermediate devices to the patient. A contrast controller may also interact with or through a computing device to adjust the contrast of images, video, or data received from the detection unit or imager unit. For example, the contrast controller may adjust the detected temperature range or the scale of the temperature ranges. Further to this example, the ranges may be adjusted from a linear scale to an exponential scale or other scales as desired by the user or medical professional.

Step 809 is alerting a user through a user interface that a thermal condition has been detected. In at least one embodiment, the thermal condition is a change in temperature or thermal gradient. Some examples may include a display as part of the user interface.

In some examples, the alert may be provided to a display that can allow medical professionals or other users to review the data provided and act on the information accordingly. In at least one embodiment, when the computing device has analyzed, determined, or processed the data or information, it can be utilized to alert doctors or other medical professionals through an alarm or display. For example, a medical professional, upon receiving the alert, may want to have a computing device or controller modify the settings of a pump that is actively providing the fluid being perfused within the organ or tissue. One example of a pump can be a Quest Medical^{®} MPS3 Myocardial Protection System or similar systems, which can include monitoring of the temperature of the fluid being provided to the patient, multiple pumping units within the pump to allow for fluid and blood to be pumped through the pump in at least one example.

In at least one example, temperature gradients or changes are utilized to ascertain cardioplegia (CPG) distribution for effective perfusion of the myocardium. The determination of the cardioplegia distribution allows for intra-operative decisions on cardioplegia delivery based on routes of delivery such as but not limited to antegrade, retro or simulgrade, flow and pressure rates or time of delivery to achieve optimal distribution to ensure optimal protection of the heart. This allows users or medical professionals to customize cardioplegia delivery for specific underlying diseases, conditions, or patient anatomies. Users or medical professionals can utilize the presentation of images, videos, data or information, and/or the processing or analysis to provide decision assistance for the various parameters, conditions, or rules to provide to a fluid dispensing system or pump through an open loop system. For example, a representation through a temperature or heat map can allow for surface or volume distribution of a cardioplegia through a heart and allow a user or medical processional to make adjustments to the delivery, delivery method, and/or delivery positioning to provide optimal delivery. Alternatively, the fluid dispensing system or pump may be controlled or auto regulated in a closed loop system based on the various parameters, conditions, or rules. For example, the flow rate of a cardioplegia can be controlled or auto regulated in the closed loop system.

In some examples, a controller, computing device, or user interface may allow for signals, alerts, or other information to be received and acted upon to trigger additional activities or operations. In this example, a controller may receive an alert, which triggers the controller to activate additional medications or requests to a pharmacy for additional medications to be available. In yet another example, the fluid dispensing system may provide additional fluids or combinations of fluids to the pump.

The same alert that is received by the controller may also be received by an alarm or similar device that can attract the attention of a medical professional or other individual that should be alerted to a change in a detected condition. In some examples, the alert may be provided to a display that can allow medical professionals or other users to review the data provided and act on the information accordingly.

Step 810 is instructing a pump, for providing material to the organ or tissue, to adjust the amount of material provided to the organ or tissue. In at least one example, the material is a fluid, drug, or bodily fluid. For example, a medical professional, upon receiving the alert, may want to have a computing device or controller modify the settings of a pump that is actively providing the fluid being perfused within the organ or tissue. In at least one embodiment, the computing device can be utilized for a control system that may comprise the pump, fluid dispensing system, alarm, and/or controller or user interface. For example, a controller or user interface may be activated to allow for control of additional medical equipment or devices within the rooms, medical suite, or surgical suite. Similarly, the alarm may also be connected to additional medical equipment or devices within the rooms, medical suite, or surgical suite in a manner that would trigger actions, steps, or operations based on specific rules, alerts, or notifications, including to the pump or a fluid dispensing system. These rules may include generating alerts or notifications when a temperature passes a specified or established threshold, for example, a specified threshold may be the desired values and the established threshold may be critical values established through medical research. Other rules may include alerts or notifications, when the temperature change does or does not occur over a specified or established period of time. Yet another rule, can include, if a temperature is above or below a specified or established threshold for specified or established period of time. These temperatures or changes in temperature may occur at discrete points of time or be an average over a period of time. If any of these rules are triggered, an alert or notification may be issued based on a determination by the computing device or other device utilized for analysis, or upon determination by a user or medical professional. In at least one example, if a rule is triggered the pump may be turned on or shutoff, in order to provide or prevent the perfusion of a cardioplegia to a heart.

For example, a medical professional, upon receiving the alert, may want to have a computing device or controller modify the settings of a pump that is actively providing the fluid being perfused within the organ or tissue. One example of a pump can be a Quest Medical^{®} MPS3 Myocardial Protection System or similar systems, which can include monitoring of the temperature of the fluid being provided to the patient, multiple pumping units within the pump to allow for fluid and blood to be pumped through the pump in at least one example. In some examples, the pump may also include information regarding the cardiac condition of a patient, such as the cardioplegia ratio, potassium, ECG, blood pressure, flow rates, and other information regarding the patient or the specific organs or tissues. The pump can receive fluids from a fluid dispensing system that can be perfused within a blood supply pumped through the pump or directly to the tissue or organ. The pump may adjust the pumping operations, perfusion, or other operations based on signals from the computing device or other instructions received from a medical professional or other user.

In some examples, the thermal imaging detection system can provide a feedback loop that allows for the control or recommended control of a pump for delivering IV, medicines, or other fluids to a patient or specific organs or tissue. In yet another example, the fluid dispensing system may provide additional fluids or combinations of fluids to the pump based on control signals receives from the computing device. The fluid dispensing system can be an IV or fluid control system that can utilize needleless connectors, manifolds, extension, or administration sets having check valves, roller clamps, filters, ball shutoffs, drop or drip control, or other administration tools. In some examples, a controller, computing device, or user interface may allow for signals, alerts, or other information to be received and acted upon to trigger additional activities or operations. In this example, a controller may receive an alert, which triggers the controller to activate additional medications or requests to a pharmacy for additional medications to be available for the pump or fluid dispensing system.

In at least one embodiment, the computing device can be utilized for a control system that may comprise the pump, fluid dispensing system, alarm, and/or controller or user interface. The control system can allow for the clinical intervention if a patient's condition does not comply with normal ranges. The clinical intervention can be activated by a doctor or medical professional, based on detected temperatures or temperature changes. The control system can provide a feedback loop of information to determine if changes made by the control system or by a user are having the desired effects or if a different intervention may be desired.

The controller or user interface may allow for connection or interaction with a pump or a fluid dispensing system. The pump or fluid dispensing system may trigger the delivery of specific fluid(s), drugs, or combinations thereof to the organ or tissue. The pump can interact with the fluid dispensing system to allow the combination, blending, or perfusion of blood, bodily fluids, drugs, bodily fluid replacements, other fluids, or combinations thereof. As the delivery of fluids occurs, then a specified zone may change to move closer to the temperature or other conditions to be monitored across all the zones. After complete perfusion or delivery of fluids, the organ or tissue may cause the temperature or other conditions to be monitored normalize across all zones.

In step 811, an updating of the imaging can occur based on automatic processing instructions or through an interaction with a user. If the user selects, or an update is done automatically 812, the method can return to step 802 or any intervening step. Alternatively, if the method is complete without out additional updates 813, the user can select to end 814.

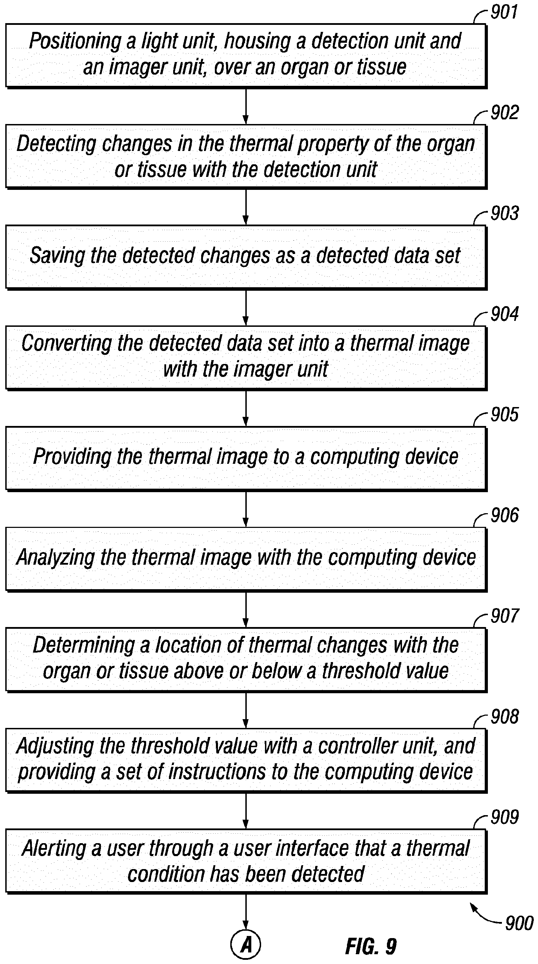

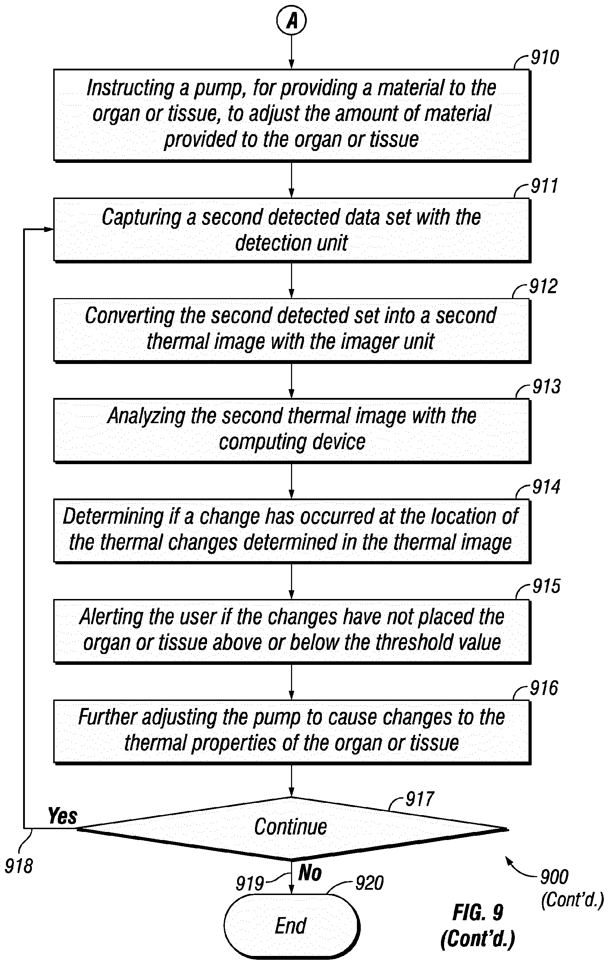

FIG. 9 is an illustration of an operational flow 900 for thermal imaging detection with a feedback loop. In step 901, a user may position a light unit above or near a patient. In at least one example, the light unit may be within an enclosure that also houses a detection unit and an imager unit over an organ or tissue of a patient. In at least one embodiment, the light unit may include multiple detection units and/or multiple imager units. The multiple detection unit(s) can be positioned in multiple locations within the surgical or medical light to allow for multi-dimensional data or data sets, such as but not limited to three-dimensional data or data sets to be generated.

In step 902, the detection unit can be utilized for detecting changes in the thermal property of the organ or tissue. In at least one example, the detection unit may be incorporated into a surgical or medical light for use in an operating room or in medical facilities. The detection unit can capture data regarding thermal changes to organs or tissues as small as 0.001 degrees Fahrenheit or its equivalent temperature in Celsius or Kelvin. In at least one example, detection can occur in a range from negative thirty (-30) degrees Fahrenheit, to one hundred twenty-five (125) degrees Fahrenheit, or equivalent temperatures in Celsius (-35 to 50 °C) or Kelvin. In some examples, the temperature range could extend from negative fifty (-50) degrees Fahrenheit, to one-hundred fifty (150) degrees Fahrenheit, or equivalent temperatures in Celsius or Kelvin. These temperature changes or gradient changes can occur prior to, during, or after a procedure or related medical operation.

The data or data sets captured, recorded, or transmitted by the detection unit may include coordinates, temperature readings, time stamps, temperature changes over time, discrete temperature, other additional data or information, comparisons, or combinations thereof. In at least one example, multiple detection unit(s) may be coupled together or multiplexed for the transmission of data or data sets to allow for multi-dimensional imaging to occur. For example, at least four detection unit(s) could be coupled or fastened to four separate points that are distal from one another along a surgical or medical light utilized in procedures or other related medical operations. Multi-dimensional imaging can allow doctors or other medical personnel to view organs and tissues at more than one level of depth or penetration to determine if perfusion or other monitored conditions or operations are occurring correctly or if changes to the procedure, operation, medicines, or other activities need to occur.

In some examples, the data or data set generated by the detection unit(s) may be stored in memory or storage as part of the detection unit(s) or transmitted in real- or near real-time. The memory or storage may be part of a system on a chip (SOC), permeant units directly coupled to the detection unit(s), or removable units that are received by the detection unit(s).

Step 903 is saving the detected changes as a detected data set. In at least one example, the detected data set can include image or video data, time and date information, and/or positioning coordinates. Data or data sets from the detection unit may be compared to the temperature readings or data from a scan prior to the procedure or medical operation that are saved in the same storage location or device. In at least one example, the data or data sets recorded or transmitted by the detection unit(s) can be provided to an imager unit directly or through an intermediary storage device. In other examples, the additional data or information may be provided by a computing device, a database connected to the computing device through a computer network, and/or other devices coupled to a computer network capable of transmitting data or information to the computing device or the imager unit.

The computing device may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation.

In step 904, an imager unit can be utilized for converting the detected data set into a thermal image. In at least one example, the imager unit can also produce video as well. In at least one embodiment, the detection unit is utilized for providing the detected thermal change to the imager unit. In some examples, the data or data sets recorded or transmitted by the detection unit(s) can be provided to an imager unit through a wired or wireless connection or through a computer network. An imager unit can be assigned or connected to an individual detection unit(s) or received a multiplexed or multiple streams of data from multiple detection units. Additionally, the detection unit(s) or imager unit may gather information regarding the ambient temperature of a room or area where the thermal image detection system is being utilized.

It should be noted that while data capture is shown as an image, the data capture may be processed by the imager unit and/or the computing device in order to provide a complete representation. In at least one example, the imager unit may be incorporated as part of the detection unit(s) or a computing device. The imager unit can take the data or data sets provided by the detection unit(s) for conversion into viewable images, video, or other displayable formats. In at least one embodiment, the imager unit can generate two- or three-dimensional images or video from the data or data sets. These images or video may be displayed through a computing device or through other devices such as, but not limited to, televisions, displays, mobile devices, tablets, phones, Personal Digital Assistants (PDAs), digital watches, smart-watches, or other devices capable of allowing viewing of information or data provided to it. In at least one example, the imager unit may also overlay additional data or information, such as, but not limited to, zones, gridlines, highlights, color maps, legends, time or date information, patient information, hospital information, doctor or medical staff information, and/or other valuable information that may be received or processed by the imager unit. In other examples, the additional data or information may be provided by a computing device, a database connected to the computing device through a computer network, and/or other devices coupled to a computer network capable of transmitting data or information to the computing device or the imager unit.

In step 905, the thermal image may be provided to a computing device. The computing device may receive the images, data, video, or other information directly or indirectly over a computer network or through a storage device such as but not limited to a hard drive, service, distributed storage, flash memory, or other storage device or combinations thereof. The computing device can be utilized to process, analyze, network, and/or communicate information or data from the various device connected, coupled, or other interfaced with the computing device.

Step 906 is analyzing the thermal image with the computing device. In at least one embodiment, the computing device creates a set of sections or zones to be reviewed for specific temperature changes or gradients. If a section or zone is outside of the specific range of temperature changes or gradients, then an alert can be generated by the computing device. Alerts may include sounds, notifications, emails, visual indicators, or other manner of capturing the attention of a medical professional.

In some examples, the temperature changes or gradients may need to be outside a specified range of temperature changes or gradients, and an alert can be generated if the temperature change or gradient is within the specified range. The computing device may also allow for the transmission of data, information, analysis, or data sets over a computer network to various databases, devices, or other computing devices, in a manner that can allow for collaboration of medical professionals before, during, and/or after a procedure or medical operation. As an example of this, a patient may have a scan performed prior to a procedure or medical operation that includes temperature readings or data for a particular organ or tissue. This set of temperature readings or data may be utilized as a baseline or initial threshold for a temperature change or gradient during an operation.

Step 907 is determining a location of thermal changes within the organ or tissue above or below a threshold value. In at least one embodiment, the location of the thermal changes may be found through zone or coordinate analysis and/or comparison to previous thermal images or data.

The data capture can be separated into zones. For example, if the data capture includes a heart or other organ or tissue, it can be presented to a user or medical professional in a three-dimensional image with vertical, horizontal, and depth zones being generated. Each of these zones can be monitored for specific thermal conditions or changes before, during, or after procedures or medical operations. In at least one embodiment, the thermal imaging detection system is utilized to provide monitoring of perfusion of drugs utilized to slow the heart during procedures or medical operations. Each zone may be set individually or in select regions for specific conditions, ranges, or other options for monitoring. For example, zones may be grouped for a heart as the areas where an artery or vessels enter and/or exit.

In at least one example, a single zone may have a significantly different temperature from adjacent zones. This can be an indication that perfusion is not occurring properly in that particular zone of the organ or tissue being monitored. The computing device can be utilized to analyze, determine, or process the data or information provided through automation, machine learning, artificial intelligence, or other computer-executed code. In at least one embodiment, when the computing device has analyzed, determined, or processed the data or information for each zone, region, or complete image or video, the computing device can be utilized to alert doctors or other medical professionals through an alarm or display.

In step 908, a controller unit can be utilized for adjusting the threshold value and providing a set of instructions to the computing device. In at least one embodiment, the controller unit can include a user interface. Doctors and medical personnel often provide fluids to patients to increase or decrease temperatures for specific operations and portions of organs or tissue. For example, during procedures or related medical operations related to the heart, fluids are perfused into the heart muscle that can slow or speed up the beating of the heart muscle, and the perfusion can be monitored by the changes in a thermal gradient of the tissue. A heart that slows down will have a temperature change or gradient difference from normal heart rhythms, in addition to the change in temperature or temperature gradient from the perfused fluids. As perfusion occurs, the temperature gradient or change in the tissue can be detected and utilized to determine if proper perfusion of medications or other fluids is occurring. If the perfusion is not occurring properly, an alarm or other indication may be provided to individuals near the procedure or related medical operation in a manner that causes individuals to monitor the patient with greater detail. In some examples, if perfusion is not occurring properly, a feedback loop of information or data can be analyzed to determine if the amount of fluid being perfused is proper or should be changed. While this can be done automatically, a doctor or other medical professional may trigger the change after reviewing a recommendation from the thermal imaging detection system.

In at least one example, the controller unit may be a user interface or other device capable of receiving interactions or inputs from a user, such as but not limited to, a doctor, nurse, or other medical professional. For example, a controller, computing device, or user interface may allow for a medical professional to input or provide preferences and/or settings for the analysis of the data, data sets, images, videos, or other information provided to the computing device. The controller or user interface may be utilized to control or receive signals from a fluid dispensing system. The fluid dispensing system can be an IV or fluid control system that can utilize needleless connectors, manifolds, extension, or administration sets having check valves, roller clamps, filters, ball shutoffs, drop or drip control, or other administration tools. In some examples, a controller, computing device, or user interface may allow for signals, alerts, or other information to be received and acted upon to trigger additional activities or operations. In this example, a controller may receive an alert, which triggers the controller to activate additional medications or requests to a pharmacy for additional medications to be available. In yet another example, the fluid dispensing system may provide additional fluids or combinations of fluids to the pump.

The same alert that is received by the controller may also be received by an alarm or similar device that can attract the attention of a medical professional or other individual that should be alerted to a change in a detected condition. In some examples, the alert may be provided to a display that can allow medical professionals or other users to review the data provided and act on the information accordingly. For example, a medical professional, upon receiving the alert, may want to have a computing device or controller modify the settings of a pump that is actively providing the fluid being perfused within the organ or tissue. In at least one embodiment, the computing device can be utilized for a control system that may comprise the pump, fluid dispensing system, alarm, and/or controller or user interface. For example, a controller or user interface may be activated to allow for control of additional medical equipment or devices within the rooms, medical suite, or surgical suite. Similarly, the alarm may also be connected to additional medical equipment or devices within the rooms, medical suite, or surgical suite in a manner that would trigger actions, steps, or operations based on specific rules, alerts, or notifications. These rules may include generating alerts or notifications when a temperature passes a specified or established threshold, for example, a specified threshold may be the desired values and the established threshold may be critical values established through medical research. Other rules may include alerts or notifications, when the temperature change does or does not occur over a specified or established period of time. Yet another rule, can include, if a temperature is above or below a specified or established threshold for specified or established period of time. These temperatures or changes in temperature may occur at discrete points of time or be an average over a period of time. If any of these rules are triggered, an alert or notification may be issued based on a determination by the computing device or other device utilized for analysis, or upon determination by a user or medical professional. In at least one example, if a rule is triggered the pump may be turned on or shutoff, in order to provide or prevent the perfusion of a cardioplegia to a heart.

The controller or user interface can also be a contrast controller to increase or decrease contrast materials to a patient or through intermediate devices to the patient. A contrast controller may also interact with or through a computing device to adjust the contrast of images, video, or data received from the detection unit or imager unit. For example, the contrast controller may adjust the detected temperature range or the scale of the temperature ranges. Further to this example, the ranges may be adjusted from a linear scale to an exponential scale or other scales as desired by the user or medical professional.

Step 909 is alerting a user through a user interface that a thermal condition has been detected. In at least one embodiment, the thermal condition is a change in temperature or thermal gradient. Some examples may include a display as part of the user interface.

In some examples, the alert may be provided to a display that can allow medical professionals or other users to review the data provided and act on the information accordingly. In at least one embodiment, when the computing device has analyzed, determined, or processed the data or information, it can be utilized to alert doctors or other medical professionals through an alarm or display. For example, a medical professional, upon receiving the alert, may want to have a computing device or controller modify the settings of a pump that is actively providing the fluid being perfused within the organ or tissue. One example of a pump can be a Quest Medical^{®} MPS3 Myocardial Protection System or similar systems, which can include monitoring of the temperature of the fluid being provided to the patient, multiple pumping units within the pump to allow for fluid and blood to be pumped through the pump in at least one example.

In at least one example, temperature gradients or changes are utilized to ascertain cardioplegia (CPG) distribution for effective perfusion of the myocardium. The determination of the cardioplegia distribution allows for intra-operative decisions on cardioplegia delivery based on routes of delivery such as but not limited to antegrade, retro or simulgrade, flow and pressure rates or time of delivery to achieve optimal distribution to ensure optimal protection of the heart. This allows users or medical professionals to customize cardioplegia delivery for specific underlying diseases, conditions, or patient anatomies. Users or medical professionals can utilize the presentation of images, videos, data or information, and/or the processing or analysis to provide decision assistance for the various parameters, conditions, or rules to provide to a fluid dispensing system or pump through an open loop system. For example, a representation through a temperature or heat map can allow for surface or volume distribution of a cardioplegia through a heart and allow a user or medical processional to make adjustments to the delivery, delivery method, and/or delivery positioning to provide optimal delivery. Alternatively, the fluid dispensing system or pump may be controlled or auto regulated in a closed loop system based on the various parameters, conditions, or rules. For example, the flow rate of a cardioplegia can be controlled or auto regulated in the closed loop system.

In some examples, a controller, computing device, or user interface may allow for signals, alerts, or other information to be received and acted upon to trigger additional activities or operations. In this example, a controller may receive an alert, which triggers the controller to activate additional medications or requests to a pharmacy for additional medications to be available. In yet another example, the fluid dispensing system may provide additional fluids or combinations of fluids to the pump.

The same alert that is received by the controller may also be received by an alarm or similar device that can attract the attention of a medical professional or other individual that should be alerted to a change in a detected condition. In some examples, the alert may be provided to a display that can allow medical professionals or other users to review the data provided and act on the information accordingly.

Step 910 is instructing a pump for providing material to the organ or tissue, to adjust the amount of material provided to the organ or tissue. In at least one example, the material is a fluid, drug, or bodily fluid. For example, a medical professional, upon receiving the alert, may want to have a computing device or controller modify the settings of a pump that is actively providing the fluid being perfused within the organ or tissue. In at least one embodiment, the computing device can be utilized for a control system that may comprise the pump, fluid dispensing system, alarm, and/or controller or user interface. For example, a controller or user interface may be activated to allow for control of additional medical equipment or devices within the rooms, medical suite, or surgical suite. Similarly, the alarm may also be connected to additional medical equipment or devices within the rooms, medical suite, or surgical suite in a manner that would trigger actions, steps, or operations based on specific rules, alerts, or notifications, including to the pump or a fluid dispensing system. These rules may include generating alerts or notifications when a temperature passes a specified or established threshold, for example, a specified threshold may be the desired values and the established threshold may be critical values established through medical research. Other rules may include alerts or notifications, when the temperature change does or does not occur over a specified or established period of time. Yet another rule, can include, if a temperature is above or below a specified or established threshold for specified or established period of time. These temperatures or changes in temperature may occur at discrete points of time or be an average over a period of time. If any of these rules are triggered, an alert or notification may be issued based on a determination by the computing device or other device utilized for analysis, or upon determination by a user or medical professional. In at least one example, if a rule is triggered the pump may be turned on or shutoff, in order to provide or prevent the perfusion of a cardioplegia to a heart.

For example, a medical professional, upon receiving the alert, may want to have a computing device or controller modify the settings of a pump that is actively providing the fluid being perfused within the organ or tissue. One example of a pump can be a Quest Medical^{®} MPS3 Myocardial Protection System or similar systems, which can include monitoring of the temperature of the fluid being provided to the patient, multiple pumping units within the pump to allow for fluid and blood to be pumped through the pump in at least one example. In some examples, the pump may also include information regarding the cardiac condition of a patient, such as the cardioplegia ratio, potassium, ECG, blood pressure, flow rates, and other information regarding the patient or the specific organs or tissues. The pump can receive fluids from a fluid dispensing system that can be perfused within a blood supply pumped through the pump or directly to the tissue or organ. The pump may adjust the pumping operations, perfusion, or other operations based on signals from the computing device or other instructions received from a medical professional or other user.

In some examples, the thermal imaging detection system can provide a feedback loop that allows for the control or recommended control of a pump for delivering IV, medicines, or other fluids to a patient or specific organs or tissue. In yet another example, the fluid dispensing system may provide additional fluids or combinations of fluids to the pump based on control signals receives from the computing device. The fluid dispensing system can be an IV or fluid control system that can utilize needleless connectors, manifolds, extension, or administration sets having check valves, roller clamps, filters, ball shutoffs, drop or drip control, or other administration tools. In some examples, a controller, computing device, or user interface may allow for signals, alerts, or other information to be received and acted upon to trigger additional activities or operations. In this example, a controller may receive an alert, which triggers the controller to activate additional medications or requests to a pharmacy for additional medications to be available for the pump or fluid dispensing system.

In at least one embodiment, the computing device can be utilized for a control system that may comprise the pump, fluid dispensing system, alarm, and/or controller or user interface. The control system can allow for the clinical intervention if a patient's condition does not comply with normal ranges. The clinical intervention can be activated by a doctor or medical professional, based on detected temperatures or temperature changes. The control system can provide a feedback loop of information to determine if changes made by the control system or by a user are having the desired effects or if a different intervention may be desired.

The controller or user interface may allow for connection or interaction with a pump or a fluid dispensing system. The pump or fluid dispensing system may trigger the delivery of specific fluid(s), drugs, or combinations thereof to the organ or tissue. The pump can interact with the fluid dispensing system to allow the combination, blending, or perfusion of blood, bodily fluids, drugs, bodily fluid replacements, other fluids, or combinations thereof. As the delivery of fluids occurs, then a specified zone may change to move closer to the temperature or other conditions to be monitored across all the zones. After complete perfusion or delivery of fluids, the organ or tissue may cause the temperature or other conditions to be monitored normalize across all zones.

Step 911 is capturing a second detected data set with the detection unit. In at least one example, multiple detected data sets may be saved in a local storage unit within the detection unit. The capturing can occur in a similar manner as step 903. Additionally, the second detected data set might be compared to the previously detected data set.

Step 912 is converting the second detected data set into a second thermal image with the imager unit. The conversion may be performed in a manner similar to that illustrated in step 904. While the conversion may be performed in a similar manner, different conversion operations, formats, codecs, or other resources may be utilized to generate similar additional thermal images, videos, information, or data.

Step 913 is analyzing the second thermal image with the computing device. In at least one embodiment, the analysis includes a comparison of the first thermal image and the second thermal image. The analysis of the second thermal image can be performed in a manner similar to step 906. However, additional analysis or comparison to the previous data may be utilized.

Step 914 is determining if a change has occurred at the location of the thermal changes determined in the thermal image. Like the determination of step 907, changes may be reviewed to ensure that changes are occurring in the proper manner.

Step 915 is alerting the user if the changes have not placed the organ or tissue above or below the threshold value. The alert of step 915 may be triggered and/or activated by similar conditions or activities as step 909. Alternatively, step 915 may have additional conditions, rules, or procedures that may be utilized to alert a user that further analysis or intervention may be required.

Step 916 is further adjusting the pump to cause changes to the thermal properties of the organ or tissue. The pump or other fluid dispensing systems may be continually adjusted based on the analysis or alerts from the thermal imaging detection system in a manner similar to that described for step 910.

In step 917, the operations can continue with a third detection and thermal image by returning 918 to step 911 or other intervening steps. Alternatively, the operations can terminate 919. At the end 920, there may be additional saving, displaying, transmission, or other operations.

The present disclosure may include a computing device that can include any of an application-specific integrated circuit (ASIC), a microprocessor, a microcontroller, a digital signal processor (DSP), a field-programmable gate array (FPGA), or equivalent discrete or integrated logic circuitry. In some examples, the system may include multiple components, such as any combination of one or more microprocessors, one or more microcontrollers, one or more DSPs, one or more ASICs, or one or more FPGAs. It would also be understood that multiples of the circuits, processors, or controllers could be used in combination or in tandem, or multithreading. Additionally, it would be understood that a browser or program could be implemented on a mobile device or mobile computing device, such as a phone, a mobile phone, a cell phone, a tablet, a laptop, a mobile computer, a Personal Digital Assistant ("PDA"), a processor, a microprocessor, a microcontroller, or other devices or electronic systems capable of connecting to a user interface and/or display system. A mobile computing device or mobile device may also operate on or in the same manner as the computing device disclosed herein or be based on improvements thereof.

Similarly, a computing device or other electrical devices described herein may be coupled through a computer network such as but not limited to, a Local Area Network (LAN), or Wireless or Wide Area Network (WLAN). The computer network may also be coupled to an intranet or the Internet to allow for voice, data, packet, or other forms of transmission between two points. It would be understood that a computer network may also include modems, routers, switches, firewalls, servers, other computing devices, and/or other transmission or transmission control devices to complete a point-to-point transmission. Moreover, the point-to-point transmission may be completed through a single node, router, server, or other transmission devices, or may pass through multiple nodes that may comprise multiple types or forms of communication protocols or transmissions means, and is not limited to telephone lines, cellular connection, Wi-Max, 3G, 4G, 5G, LTE cellular services, cable, fiber, or giga-fiber.

The components of the present disclosure may include any discrete and/or integrated electronic circuit components that implement analog and/or digital circuits capable of producing the functions attributed to the modules herein. For example, the components may include analog circuits, e.g., amplification circuits, filtering circuits, and/or other signal conditioning circuits. The components may also include digital circuits, e.g., combinational or sequential logic circuits, memory devices, etc. Furthermore, the modules may comprise memory that may include computer-readable instructions that, when executed, cause the modules to perform various functions attributed to the modules herein.

Memory may include any volatile, non-volatile, magnetic, or electrical media, such as a random access memory (RAM), dynamic random access memory (DRAM), static random access memory (SRAM), read-only memory (ROM), non-volatile RAM (NVRAM), electrically-erasable programmable ROM (EEPROM), flash memory, hard disks, or any other digital media. Additionally, there may also be a tangible non-transitory computer-readable medium or media that contains machine instructions, such as a (portable or internally installed) hard drive disc, a flash drive, a compact disc, a DVD, a zip drive, a floppy disc, optical medium, magnetic medium, or any other number of possible drives or discs, that are executed by the internal logic of a computing device. It would be understood that the tangible non-transitory computer-readable medium could also be considered a form of memory or storage media.

While videos and images are described herein, it would be understood that any encoding, compression, format, formatting, conversion, codec, style, containers, or storage manner can be utilized without departing from the spirit of the present disclosure. Video encoding, compression containers, codecs, or formats that may be utilized with the present disclosure may comprise, but are not limited to, Moving Pictures Experts Group (MPEG) or its variations (H.264, MPEG-1, MPEG-2, MPEG-3, MPEG-4, MP3, or MPR), MOV or a QuickTime Movie, Windows Media Video (WMV), Flash Video (FLV), Audio-Video Interleaved (AVI), Advanced Video Coding High Definition (AVCHD), Web Media (WebM), Matroska Multimedia (MKV), further developed encoding, compression, format, formatting, conversion, codec, style, containers, or storage manner, or combinations thereof. Image encoding, compression, format, formatting, conversion, codec, style, containers, or storage manners that may be utilized with the present disclosure may comprise, but are not limited to, Animated Portable Network Graphics (APNG), AV1 Image File Format (AVIF), Graphics Interchange Format (GIF), Joint Photographic Expert Group image (JPEG), Portable Network Graphics (PNG), Scalable Vector Graphics (SVG), Web Picture Format (WebP), Bitmap File (BMP), Microsoft Icon (ICO), Tagged Image File Format (TIFF), RAW, further developed encoding, compression, format, formatting, conversion, codec, style, containers, or storage manner, or combinations thereof.

While this disclosure has been particularly shown and described with reference to preferred embodiments, it will be understood by those skilled in the art that various changes in form and detail may be made therein without departing from the spirit and scope of the invention. The inventors expect skilled artisans to employ such variations as appropriate, and the inventors intend the invention to be practiced otherwise than as specifically described herein. Accordingly, this disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

While various embodiments in accordance with the principles disclosed herein have been described above, it should be understood that they have been presented by way of example only and not limitation. Thus, the breadth and scope of this disclosure should not be limited by any of the above-described exemplary embodiments but should be defined only in accordance with any claims and their equivalents issuing from this disclosure. Furthermore, the above advantages and features are provided in described embodiments but shall not limit the application of such issued claims to processes and structures accomplishing any or all of the above advantages.

Additionally, the section headings herein are provided for consistency with the suggestions under 37 C.F.R. 1.77 or otherwise to provide organizational cues. These headings shall not limit or characterize the invention(s) set out in any claims that may issue from this disclosure. Specifically, and by way of example, although the headings refer to a "Technical Field," the claims should not be limited by the language chosen under this heading to describe the so-called field. Further, a description of technology as background information is not to be construed as an admission that certain technology is prior art to any embodiment(s) in this disclosure. Neither is the "Brief Summary" to be considered as a characterization of the embodiment(s) set forth in issued claims. Furthermore, any reference in this disclosure to "invention" in the singular should not be used to argue that there is only a single point of novelty in this disclosure. Multiple embodiments may be set forth according to the limitations of the multiple claims issuing from this disclosure, and such claims accordingly define the embodiment(s), and their equivalents, that are protected thereby. In all instances, the scope of such claims shall be considered on their own merits in light of this disclosure but should not be constrained by the headings set forth herein.

## Claims

1. A system for detecting the uniformity of temperature of a tissue during perfusion of a substance into the tissue, the system comprising:
(a) a thermal detector;
(b) an imager to provide image data from the thermal detector of a temperature for the tissue being imaged.

2. The system of claim 1 further comprises:
(c) a contrast controller to provide increased contrast between imaged areas having different temperatures.

3. The system of claim 1 or of claim 2 further comprises:
(c) an alarm to indicate if a detected temperature or temperature change difference exceeds a predefined amount; and/or
(c) an alarm to indicate if a detected temperature or temperature change is outside of a predefined range.

4. The system of claim 1 or of claim 2 or of claim 3 further comprises:
(c) a lighting source holding the thermal detector above the tissue; and/or
(c) a control system for communicating with a pump delivering the substance into the tissue.

5. The system of claim 1 or of any preceding claim further comprises:
(c) a control system with a set of clinical interventions that provides decision options to the clinician for appropriate interventions based on detected temperatures and temperature changes.

6. The system of claim 1 or of any preceding claim further comprises:
(c) an imaging algorithm that detects changes in temperature over time compared to a standard and provides feedback on differences from that standard; and/or
(c) an imaging algorithm that detects changes in temperature between adjacent tissues and provides feedback on differences to those changes based on predefined ranges; and optionally or preferably wherein a set of zones within the image data are detected with the imaging algorithm that provides feedback on differences of those changes based on predefined ranges.

7. The system of claim 1 or of any preceding claim, wherein the thermal detector is housed in a handheld unit, and optionally or preferably further comprising:
(c) an adaptor for mounting the handheld unit to a mounting location, and further optionally or preferably wherein the mounting location is a specialized pole for use in a medical facility.

8. The system of claim 5 or any claim dependent directly or indirectly from claim 5, wherein the appropriate interventions comprise automatic delivery of fluids to the tissue based on detected temperatures and temperature changes.

9. An apparatus for detecting thermal changes comprising:
(a) a thermal detector; and
(b) an imager to provide image data from a thermal detector of a temperature for the perfused tissue being imaged.

10. The apparatus of claim 9 further comprising:
(c) a housing holding the thermal detector and the imager; and/or wherein the thermal detector and imager are connected to a computing device.

11. The apparatus of claim 10, wherein the housing further comprising a light source, and optionally or preferably wherein the light source provides light to a surgical or doctor supervised operation.

12. The apparatus of claim 10 or of claim 11, wherein the computing device executing a set of operational commands for an imaging algorithm, and optionally or preferably wherein the imaging algorithm detects the changes in temperatures for a set of zones of the perfused tissue.

13. The apparatus of claim 9 or of any of claims 10 to 12 further comprises an alarm that is activated when the temperature goes outside of a predefined range.

14. A method for imaging tissue comprising:
detecting a thermal gradient with a thermal detector;
imaging a set of data generated by the thermal detector; and
determining if the thermal gradient exceeds a predefined range.

15. The method of claim 14, wherein the thermal detector is coupled to a computing device utilizing an imaging algorithm to determine the changes in the thermal gradient within zones of a tissue through which a fluid is perfused, or is to be perfused.

16. The method of claim 14 or of claim 15, further comprising:
(i) providing inter-operative decision assistance if the thermal gradient meets specific conditions; and/or
(ii) displaying the image of the set of data as a heat map.
